(19) Europäisches Patentamt
European Patent Office
Office européen des brevets

(11) EP 2 770 982 B1

(12) **EUROPEAN PATENT SPECIFICATION**

(45) Date of publication and mention of the grant of the patent:
**19.04.2017 Bulletin 2017/16**

(21) Application number: **12780395.5**

(22) Date of filing: **24.10.2012**

(51) Int Cl.:
***A61K 9/20*** *(2006.01)* ***A61K 9/00*** *(2006.01)*
***A61K 31/135*** *(2006.01)*

(86) International application number:
**PCT/US2012/061644**

(87) International publication number:
**WO 2013/063082 (02.05.2013 Gazette 2013/18)**

(54) **IMPLANTABLE RASAGILINE COMPOSITIONS AND METHODS OF TREATMENT THEREOF**

IMPLANTIERBARE RASAGILINZUSAMMENSETZUNGEN UND VERFAHREN ZUR BEHANDLUNG

COMPOSITIONS DE RASAGILINE IMPLANTABLES ET MÉTHODES DE TRAITEMENT ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2011 US 201161550653 P**
**08.08.2012 US 201261680913 P**

(43) Date of publication of application:
**03.09.2014 Bulletin 2014/36**

(73) Proprietor: **Braeburn Pharmaceuticals, Inc.**
**Princeton, NJ 08542 (US)**

(72) Inventors:
- **SCHWARZ, Alexander**
**Brookline, MA 02446 (US)**
- **DECKER, Stefanie**
**Princeton, NJ 08540 (US)**

(74) Representative: **RatnerPrestia**
**Altheimer Eck 2**
**80331 München (DE)**

(56) References cited:
**WO-A1-2010/039717** **WO-A1-2010/039722**
**WO-A1-2011/116132** **US-A- 5 035 891**
**US-A1- 2009 098 182**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 770 982 B1

**Description**

FIELD OF THE INVENTION

The invention relates to reservoir-based drug delivery compositions that are implantable into a subject in order to deliver therapeutically effective amounts of rasagiline at a pseudo-zero order rate, for extended periods of time (e.g., at least one month, one year, etc.).

BACKGROUND OF THE INVENTION

Drug compositions come in many different forms and may be administered to a patient via several different routes, such as oral, parenteral, topical, intravenous, subcutaneous, intranasal, etc. Depending on the active and the treatment desired, different routes of administration may be preferable.

Some diseases and conditions may be long lasting, requiring treatment for many weeks, months, or even years. Typically, a patient taking a traditional oral dosage form (*e.g*,, tablets or capsules) may be required to take the oral dose at least once per day for the duration of the treatment. For example, a patient may need to take an oral dose twice a day for a year or longer. The problem with treatments that require continuous dosage over a long period of time is that often the patient may not be compliant in taking the medications. In other words, the patient may forget, believe the treatment is unnecessary, or grow tired of having to take many pills over an extremely long period of time. Accordingly, treatments are necessary which can alleviate these compliance issues, but still provide effective and efficient treatment to the patient.

Parkinson's disease is a progressive neurodegenerative disorder that affects more than one million people in the United States, including 1% of the population over the age of 55. Parkinson's disease is characterized by a patient's selective loss of dopaminergic neurons, which results in motor impairments, such as bradykinesia (i.e., slowness of movement), tremors, muscular rigidity, and postural instability. Treatment of the symptoms of Parkinson's disease typically focuses on the replacement or augmentation of dopamine. This is often achieved through the administration of dopamine receptor agonists, or the dopamine precursor levodopa. Monoamine oxidase B (MAO-B) inhibitors, such as rasagiline and selegiline, provide an alternative first-line treatment for the symptoms of Parkinson's disease, or serve as an adjunctive treatment in addition to other drugs, such as levodopa. As Parkinson's disease progresses, motor complications, including "wearing off," may occur. "Wearing off " is a phenomenon characterized by periods of decreasing effectiveness of medication, causing symptoms to re-emerge before the next dose, including, for example, motor symptoms (e.g., tremor and problems with balance), non-motor symptoms (e.g., anxiety, fatigue, mood changes, and restlessness), and autonomic nervous system dysfunction (e.g., sweating and hypersalivation). Treatment of the symptoms of Parkinson's disease typically lasts many years, i.e., for the rest of a patient's life.

Accordingly, there has remained a need for effective dosage forms that provide therapeutically effective amounts of drugs that treat the symptoms of Parkinson's disease at relatively constant rates over a long period of time.

SUMMARY OF THE INVENTION

Aspects of the present invention include reservoir-based drug delivery compositions, which may be implanted into a subject in order to deliver a therapeutically effective amount of rasagiline to the subject for long periods of time (*e.g.,* at least one month at least one month, at least six months, at least one year, at least 18 months, at least two years, at least 30 months, etc.). The therapeutically effective amount of rasagiline may be delivered at a pseudo-zero order rate (e.g., zero order rate). Accordingly, the present invention is directed to rasagiline compositions, methods of treatment (*e.g.,* treating one or more symptoms of Parkinson's disease), methods of delivering rasagiline, subcutaneous delivery systems, and kits regarding the same.

According to another embodiment of the present invention, a drug delivery composition comprises a drug elution rate-controlling excipient comprising an elastomeric polymer defining a reservoir, and the reservoir contains at least one discrete solid dosage form comprising rasagiline hemitartrate. The drug delivery composition is in an implantable dosage form. According to one aspect of the present invention, the at least one discrete solid dosage form comprises 75-97 wt% (*e.g.,* about 88 wt%) rasagiline hemitartrate based on the total weight of the at least one discrete solid dosage form and 1-25 wt% (*e.g.,* about 10 wt%) of at least one sorption enhancer based on the total weight of the at least one discrete solid dosage form.

According to another embodiment of the present invention, a method of treating symptoms of Parkinson's disease comprises implanting a reservoir-based drug delivery composition into a subject to systemically deliver a therapeutically effective amount of rasagiline to the subject for a period of time of at least one month. The drug delivery composition may comprise at least one discrete solid dosage form comprising rasagiline hemitartrate surrounded by an excipient comprising at least one polymer. The therapeutically effective amount of the rasagiline may be delivered at a

pseudo-zero order rate (e.g., zero order rate). The at least one discrete solid dosage form may comprise 75-97 wt% (*e.g.,* about 88 wt%) rasagiline hemitartrate based on the total weight of the at least one discrete solid dosage form and 1-25 wt% (*e.g.,* about 10 wt%) of at least one sorption enhancer based on the total weight of the at least one discrete solid dosage form.

According to another embodiment of the present invention, a method of systemically delivering rasagiline to a subject includes releasing a therapeutically effective amount of rasagiline from a reservoir-based composition comprising a polymeric rate-controlling excipient defining a reservoir containing at least one discrete solid dosage form comprising rasagiline hemitartrate to provide a pseudo-zero order elution rate (e.g., zero order rate) to the subject for a period of time of at least one month.

According to another embodiment of the present invention, a drug delivery composition comprises a drug elution rate-controlling excipient comprising an elastomeric polymer defining a reservoir, and the reservoir contains at least one discrete solid dosage form comprising rasagiline hemitartrate.

According to another embodiment of the present invention, a subcutaneous delivery system comprises an elastomeric reservoir implant comprising at least one discrete solid dosage form surrounded by a polymeric rate-controlling excipient. The at least one discrete solid dosage form comprises rasagiline hemitartrate. The subcutaneous delivery system provides for release of the rasagiline at an elution rate suitable to provide a therapeutically effective amount of the rasagiline to a subject at a pseudo-zero order rate for a period of time of at least one month.

According to another embodiment of the present invention, a kit for subcutaneously placing a drug delivery composition comprises a reservoir-based drug delivery composition comprising a polymeric rate-controlling excipient defining a reservoir containing at least one discrete solid dosage form comprising rasagiline hemitartrate; and an implanter for inserting the reservoir-based drug delivery composition beneath the skin, and optionally instructions for performing the implantation and explantation of the drug delivery composition.

According to another embodiment of the present invention, a method of delivering a therapeutically effective amount of rasagiline from an implantable drug delivery composition comprises implanting a reservoir-based drug delivery composition into a subject to systemically deliver a therapeutically effective amount of rasagiline to the subject at a pseudo-zero order rate for a period of time of at least one month. The drug delivery composition comprises at least one discrete solid dosage form surrounded by an excipient comprising at least one polymer, and the at least one discrete solid dosage form comprises rasagiline hemitartrate. The polymer comprises a substantially non-porous, elastomeric polymer comprising soft and hard segments, and the relative content of the soft and hard segments provide an elution rate within a target range of average daily elution rate for the rasagiline.

According to another embodiment of the present invention, a drug delivery composition includes a rate-controlling excipient defining a reservoir which contains at least one discrete solid dosage form comprising rasagiline hemitartrate. The rate-controlling excipient comprises a substantially non-porous, elastomeric polymer comprising soft and hard segments selected based on the relative content of soft and hard segments of the polymer to obtain an elution rate within a target range of average daily elution rate for the rasagiline. The at least one discrete solid dosage form comprises at least one sorption enhancer in an amount effective to modulate the average daily elution rate of the rasagiline to provide for release of the rasagiline at pseudo-zero order within the target range at the therapeutically effective amount for a period of time of at least one month. The amount of sorption enhancer is preferably directly proportional to the average daily elution rate. The rasagiline hemitartrate composition preferably delivers a therapeutically effective amount of rasagiline to a subject at a target range of about 100 micrograms/day to about 1000 micrograms/day, for example 700 micrograms/day.

According to another embodiment of the present invention, a subcutaneous delivery system for releasing rasagiline at a pseudo-zero order comprises an elastomeric reservoir implant comprising a rate-controlling excipient defining a reservoir. The rate-controlling excipient comprises a substantially non-porous elastomeric polymer having a relative content of hard segments and soft segments to provide an elution rate within a target range of average daily elution rate for the rasagiline. The reservoir contains at least one discrete solid dosage form comprising rasagiline hemitartrate and an effective amount of at least one sorption enhancer to modulate the elution rate of the rasagiline for release of a therapeutically effective amount of the rasagiline within the target range at pseudo-zero order for a period of time of at least one month. The amount of sorption enhancer may be directly proportional to the average daily elution rate.

According to another embodiment of the present invention, a method of choosing an implantable drug delivery composition comprises selecting a rate-controlling excipient comprising a substantially non-porous, elastomeric polymer comprising soft and hard segments for defining a reservoir based on the relative content of soft and hard segments of the polymer to adjust the elution rate within a target range of average daily elution rate for rasagiline; and selecting and formulating rasagiline hemitartrate and at least one sorption enhancer in order to modulate the elution rate at a therapeutically effective amount of the rasagiline at pseudo-zero order for a period of time of at least one month, wherein the amount of sorption enhancer is directly proportional to the average daily elution rate.

According to another embodiment of the present invention, a method of making an implantable drug delivery

composition includes: (a) selecting a substantially non-porous elastomeric polymer comprising soft and hard segments based on the relative content and molecular weights of the soft and hard segments of the polymer to provide an elution rate within a target range of average daily elution rate for rasagiline; (b) forming a hollow tube from the elastomeric polymer (see *e.g.,* Figure 2); (c) selecting and formulating rasagiline hemitartrate and at least one sorption enhancer in order to produce an elution rate at a therapeutically effective amount of rasagiline at pseudo-zero order for a period of time of at least one month, wherein the amount of sorption enhancer is directly proportional to the average daily elution rate; (d) loading at least one discrete solid dosage form comprising the rasagiline hemitartrate and the at least one sorption enhancer into the tube; and (e) sealing both ends of the tube to form a sealed cylindrical reservoir-based drug delivery composition.

## BRIEF DESCRIPTION OF THE DRAWINGS

The invention may be further understood by reference to the drawings in which:

Figure 1 depicts the role of the excipient in a reservoir-based drug delivery composition according to one aspect of the present invention;
Figure 2 depicts the cylindrical shape of a reservoir-based drug delivery composition according to one embodiment of the present invention;
Figure 3 depicts the difference between a drug reservoir and a matrix-based implant;
Figure 4 is a graph showing the *in vitro* elution rate (μg/day) of rasagiline from implants of the present invention comprising rasagiline hemitartrate over about 21 weeks, according to embodiments of the present invention described in Example 2;
Figure 5 is a graph showing the *in vitro* elution rate (μg/day) of rasagiline from implants of the present invention comprising rasagiline mesylate over about 10 weeks, according to embodiments of the present invention described in Example 3;
Figure 6 is a graph showing the *in vitro* elution rate (μg/day) of rasagiline from implants of the present invention comprising rasagiline hemitartrate over about 31 weeks, according to embodiments of the present invention described in Example 4;
Figure 7 is a graph of the average *in vivo* plasma concentrations (ng/mL) of rasagiline in beagle dogs that were implanted with rasagiline hemitartrate implants of the present invention, according to embodiments of the present invention described in Example 5;
Figure 8 is a perspective view of a kit for subcutaneously placing a drug-eluting implant in a subject according to embodiments of the invention;
Figure 9 is a perspective view of an insertion instrument used in the kit of Figure 8;
Figure 9A is a cross-sectional view about section line A-A in Figure 9;
Figure 10 is another perspective view of the insertion instrument of Figure 8;
Figure 11 is a distal end view of the insertion instrument of Figure 8;
Figure 12 is a proximal end view of the insertion instrument of Figure 8;
Figure 13 is a side elevation view of the insertion instrument of Figure 8;
Figure 14 is another side elevation view of the insertion instrument of Figure 8;
Figure 15 is a top plan view of the insertion instrument of Figure 8;
Figure 16 is a bottom plan view of the insertion instrument of Figure 8;
Figure 17 is a cross-sectional view about section line B-B in Figures 10 and 15 of the insertion instrument of Figure 8;
Figure 18 is a perspective view of another kit for subcutaneously placing a drug-eluting implant in a subject, according to another aspect of the invention;
Figure 19 is a side elevation view of a tunneling instrument used in the kit of Figure 18;
Figure 20 is another side elevation view of the tunneling instrument of Figure 18;
Figure 21 is a perspective view of the tunneling instrument of Figure 18;
Figure 22 is another perspective view of the tunneling instrument of Figure 18;
Figure 23 is a top plan view of the tunneling instrument of Figure 18;
Figure 24 is a bottom view of the tunneling instrument of Figure 18;
Figure 25 is a cross-sectional view about section line C-C in Figures 22 and 23 of the tunneling instrument of Figure 18;
Figure 26 is a distal end view of the tunneling instrument of Figure 18;
Figure 27 is a proximal end view of the tunneling instrument of Figure 18.

## DETAILED DESCRIPTION OF THE INVENTION

Aspects of the present invention include methods of treatment, such as methods of treating symptoms of

Parkinson's disease; methods of delivering rasagiline from an implantable composition in a therapeutically effective amount to a patient; reservoir-based rasagiline delivery compositions; subcutaneous delivery systems for rasagiline; and kits for subcutaneous delivery of rasagiline.

As used herein, the term "therapeutically effective amount" refers to those amounts that, when administered to a particular subject in view of the nature and severity of that subject's disease or condition, will have a desired therapeutic effect, *e.g.,* an amount which will cure, prevent, inhibit, or at least partially arrest, delay the onset of or partially prevent a target disease or condition or one or more symptoms thereof.

The terms "active pharmaceutical ingredient," "API," "drug," or "active" may be used herein interchangeably to refer to the pharmaceutically active compound(s) in the drug delivery composition. This is in contrast to other ingredients in the drug delivery composition, such as excipients, which are substantially or completely pharmaceutically inert. The API in exemplary embodiments of the present invention is rasagiline hemitartrate.

The term "pharmaceutically acceptable," as used herein, means approved by a regulatory agency, *e.g.* of the U.S. Federal or a state government or listed in the U.S. Pharmacopeia or other generally recognized pharmacopeia for use in animals, and more particularly in humans.

Each compound used herein may be discussed interchangeably with respect to its chemical formula, chemical name, abbreviation, etc. For example, PTMO may be used interchangeably with poly(tetramethylene oxide). Additionally, each polymer described herein, unless designated otherwise, includes homopolymers, copolymers, terpolymers, and the like.

As used herein and in the claims, the terms "comprising" and "including" are inclusive or open-ended and do not exclude additional unrecited elements, compositional components, or method steps. Accordingly, the terms "comprising" and "including" encompass the more restrictive terms "consisting essentially of" and "consisting of." Unless specified otherwise, all values provided herein include up to and including the endpoints given, and the values of the constituents or components of the compositions are expressed in weight percent of each ingredient in the composition.

Treatment of Parkinson's Disease

Parkinson's disease is a progressive neurodegenerative disorder that is characterized by a patient's loss of dopaminergic neurons, which results in motor impairments, such as bradykinesia (i.e., slowness of movement), tremors, muscular rigidity, and postural instability. The majority of pharmacological therapies used for the management of symptoms of Parkinson's disease have focused on restoring dopamine in the striatal region of the brain by administering the dopamine precursor levodopa, or by administering dopamine receptor agonists.

Monoamine oxidase B (MAO-B) inhibitors, such as rasagiline, provide an alternative first-line treatment for the symptoms of Parkinson's disease, or serve as an adjunctive treatment in addition to other drugs, such as levodopa. MAO, a flavin-containing enzyme, is classified into two major molecular species, A and B, and is localized in mitochondrial membranes throughout the body in nerve terminals, brain, liver, and intestinal mucosa. MAO regulates the metabolic degradation of catecholamines and serotonin in the central nervous system and peripheral tissues. MAO-B is the major form in the human brain and is involved in changing active dopamine to its inactive catabolites.

MAO-B inhibitors that are approved for use include selegiline (Eldepryl®, zelapar®) and rasagiline (Azilect®). One of the drawbacks of selegiline is its metabolism to amphetamines, which may produce neurotoxic or adverse cardiovascular effects. In contrast, rasagiline has no amphetamine metabolites. In *ex vivo* animal studies in brain, liver, and intestinal tissues, rasagiline has shown to be a potent, irreversible MAO-B selective inhibitor. One mechanism of action of rasagiline is believed to be its MAO-B inhibitory activity, which causes an increase in extracellular levels of dopamine in the brain.

Treatment of one or more of the symptoms of Parkinson's disease according to embodiments of the present invention include treatment of one or more symptoms known to one of ordinary skill in the art. Symptoms of Parkinson's disease may include, but are not limited to, motor impairments such as bradykinesia (i.e., slowness of movement), problems with balance, muscular rigidity, postural instability, and/or tremors. Symptoms of Parkinson's disease may also include, but are not limited to, non-motor symptoms, such as bladder and bowel dysfunction, postural hypotension, anxiety, apathy, dementia, depression, psychosis, pain, and/or sleep disturbances.

The treatment of one or more of the symptoms of Parkinson's disease can require long-lasting treatment, often on the order of many years. The treatment of symptom(s) of Parkinson's disease in accordance with the present invention is directed to early or advanced Parkinson's disease, and to monotherapy (i.e., as a subject's only dopaminergic medication) or adjunctive therapy (i.e., used in addition to (with or after) treatment with one or more other dopaminergic medications, typically levodopa). When the treatment is used as monotherapy, the treatment may comprise the patient's initial or "first-line" dopaminergic therapy. It is believed that when rasagiline is used as a monotherapy, it primarily inhibits catabolism of endogenous dopamine, whereas when rasagiline is used in combination with levodopa, it also inhibits catabolism of exogenous dopamine.

By "treatment," it is intended that a pharmaceutically effective amount of rasagiline would be administered via

the drug delivery composition, which will inhibit, or at least partially arrest or partially prevent or suppress one or more symptoms of Parkinson's disease. For example, treatment may include treatment that can suppress one or more motor impairments, such as bradykinesia, muscular rigidity, postural instability, and/or tremors. The treatment is particularly effective in that once the implant is administered to the patient, the patient will continue to receive a therapeutically effective dose for the intended duration of the implant (*e.g.,* one month, three months, six months, one year, 18 months, two years, 30 months, or more). This is in contrast to the oral dose, which requires compliance by the patient and continued oral administration consistently over the same duration of time.

According to one aspect of the present invention, a method of treating one or more symptoms of Parkinson's disease comprises implanting a reservoir-based drug delivery composition into a subject to systemically deliver a therapeutically effective amount of rasagiline to the subject for a period of time of at least one month. The drug delivery composition comprises at least one discrete solid dosage form comprising rasagiline hemitartrate surrounded by an excipient comprising at least one polymer.

According to another aspect of the present invention, a method of systemically delivering rasagiline to a subject includes releasing a therapeutically effective amount of rasagiline from a reservoir-based composition comprising a polymeric rate-controlling excipient defining a reservoir containing at least one discrete solid dosage form comprising rasagiline hemitartrate to provide a pseudo-zero order elution rate (e.g., zero order rate) to the subject for a period of time of at least one month.

According to another embodiment, a drug delivery composition comprises a drug elution rate-controlling excipient comprising an elastomeric polymer defining a reservoir. The reservoir contains at least one discrete solid dosage form comprising rasagiline hemitartrate, and the drug delivery composition is in an implantable dosage form. The reservoir preferably contains at least one discrete solid dosage form comprising 75-97 wt% rasagiline hemitartrate based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. The composition preferably delivers a therapeutically effective amount of rasagiline to a subject at a target range of about 100 micrograms/day to about 1000 micrograms/day.

<u>Salt Forms of Rasagiline</u>

Rasagiline mesylate is currently on the market in the form of tablets for oral use (Azilect®), and is indicated for the treatment of the signs and symptoms of Parkinson's disease as initial monotherapy, and as adjunct therapy to levodopa. Azilect® is administered once daily at a dose of 1 mg, or, as an adjunct to levodopa, 0.5 mg once daily or up to 1 mg daily as required for sufficient clinical response. The effectiveness of Azilect® has been demonstrated in patients with early Parkinson's disease who were receiving Azilect® as monotherapy and who were not receiving any concomitant dopaminergic therapy. The effectiveness of Azilect® as adjunct therapy has also been demonstrated in patients with Parkinson's disease who were treated with levodopa.

During the development of the present invention, it was discovered that when rasagiline mesylate was used as the API salt in the implantable drug delivery compositions, the elution rate *in vitro* started high and then dropped consistently over the following 9 weeks (*see e.g,* Example 3 below and Figure 5). By week 4, the implants had begun to swell, and between week 9 and week 10, the implants ruptured and burst. Thus, although rasagiline mesylate has consistently been the preferred salt form for oral dosage forms of rasagiline, it did not prove to be a suitable salt form when placed in implantable drug delivery compositions of the present invention. However, the applicant surprisingly discovered that when rasagiline hemitartrate was used as the API salt in the implantable drug delivery compositions, instead of rasagiline mesylate, the implants did not swell and burst, but instead provided pseudo-zero order release rates of rasagiline over several weeks (*see, e.g.,* Examples 2 and 4 below, and Figures 4 and 6). The applicant therefore discovered that rasagiline hemitartrate possesses unexpectedly advantageous properties, particularly in comparison to rasagiline mesylate, as a salt form of rasagiline that can be used in a new route of administration, namely, in implantable drug delivery compositions that can deliver a therapeutically effective amount of rasagiline.

<u>Reservoir-Based Drug Delivery Composition</u>

The drug delivery composition is a reservoir-based drug delivery composition. As used herein, the "reservoir-based composition" is intended to encompass a composition having a substantially or completely closed, surrounded, or encased hollow space or reservoir, where the hollow space or reservoir is filled, at least partially, with at least one discrete solid dosage form.

In one embodiment of the present invention, a drug delivery composition comprises a drug elution rate-controlling excipient comprising an elastomeric polymer defining a reservoir, and the reservoir contains at least one discrete solid dosage form comprising rasagiline hemitartrate. The elastomeric polymer defining the reservoir is formed separate from the at least one discrete solid dosage form (i.e., the elastomeric polymer defining the reservoir and the at least one

discrete solid dosage form are not two "layers" that are bonded to each other; rather, the elastomeric polymer defining the reservoir is separately formed and the at least one discrete solid dosage form is placed into contact with the elastomeric polymer when it is loaded into the reservoir).

A reservoir-based composition, as used herein, is in contradistinction to a matrix-based composition. As depicted in Figure 3, a drug reservoir includes a reservoir portion 120 and a rate controlling portion (excipient 110) whereas a matrix-based implant only consists of the matrix material 130 with the drug incorporated therein. In other words, in a reservoir system, the drug is contained within or is surrounded by some type of rate-controlling material (*e.g.,* a wall, membrane, or casing). In a matrix system, the drug is combined within some type of matrix, often polymeric, which often erodes or degrades in order to release the active to the subject.

Thus, there are some major distinctions between the two types of systems. The reservoir-based system allows for a much higher drug loading (*e.g.*, on the order of 98% maximum) whereas a matrix-based system contains a much smaller amount (*e.g.,* on the order of 25% maximum). Although a higher drug loading may be beneficial, it can also be dangerous because of the increased risk of drug overdose or dumping into the subject if the surrounding material were to break or rupture. Additionally, the reservoir-based composition of the present invention allows for a pseudo-zero order rate (e.g., zero order rate) of release of the active. A matrix-based system, on the contrary, provides for a first order rate of release. A first order rate may be characterized by a high initial rate of release that decays or diminishes quickly over time.

As used herein, the term "pseudo-zero order" or "pseudo-zero order rate" refers to a zero-order, near-zero order, substantially zero order, or controlled or sustained release of an API. A zero order release profile may be characterized by release of a constant amount of the API per unit time. A pseudo-zero order release profile may be characterized by approximating a zero-order release by release of a relatively constant amount of the API per unit time (*e.g.*, within 40%, 30%, 20%, or 10% of the average value). Under a pseudo-zero order rate, the composition may initially release an amount of the API that produces the desired therapeutic effect, and gradually and continually release other amounts of the API to maintain the level of therapeutic effect over an extended period of time (*e.g.,* at least one month, six months, one year, or more than one year). In order to maintain a near-constant level of API in the body, the API may be released from the composition at a rate that will replace the amount of API being metabolized and/or excreted from the body. It will be appreciated by one of ordinary skill in the art that there may be some initial period of time before steady state is reached (*e.g.,* a ramp up or an initial spike before the target range is reached, as shown, for example, in Figures 4 and 6 prior to about week 4), which still complies with the present definition of "pseudo-zero order."

Without wishing to be bound to a particular theory, it is believed that a concentration gradient occurs where the concentration of API within the reservoir is "infinite" (*e.g.,* the reservoir acts an infinite supply, but the concentration is practically limited by the amount of active for the given duration of release) and the concentration outside the drug delivery composition is zero (*e.g.*, the subject acts as an infinite sink where the active is constantly being taken away from the composition by the subject's body, such as circulatory, lymphatic systems, etc.). Additionally, the excipient 110 (*e.g.,* the wall through which the active passes) becomes fully saturated with the active ingredient at steady state. Accordingly, this gradient allows the "infinite" supply of API to be adsorbed into the excipient, dissolve in and diffuse through the polymer wall, and then be desorbed for release into the subject. The selection of the excipient 110 may help to provide the pseudo-zero order release of the drug. Without wishing to be limited or bound by any theory, it is believed that the release of the drug is not dependent on the desorption from the excipient.

## Dosage Form(s)

The drug delivery composition comprises at least one dosage form comprising at least one API. In one embodiment of the present invention, the drug delivery composition comprises at least one discrete solid dosage form comprising rasagiline hemitartrate surrounded by an excipient comprising at least one polymer.

As used herein, the term "discrete solid dosage form" is intended to encompass any dosage form that is in the form of a solid. The solid dosage form may include any cohesive solid form (*e.g.,* compressed formulations, pellets, tablets, etc.) The solid dosage form may include a solid body or mass comprising the API, which may be prepared in any suitable manner known to one of ordinary skill in the art (*e.g.,* compressed, pelleted, extruded).

The solid dosage forms are "discrete" in that there are one or more dosage forms contained within the reservoir. In other words, the discrete solid dosage form includes one or more solid formulations which are separate and distinct from the polymeric rate-controlling excipient. In an exemplary embodiment, the discrete solid dosage form(s) do not fill the entire reservoir or cavity (*e.g.,* the solid dosage forms are substantially cylindrical and the reservoir is substantially cylindrical). For example, the solid dosage form need not be co-extruded with the surrounding excipient such that the solid dosage form fills the entire cavity.

According to one embodiment of the present invention, the discrete solid dosage forms in the drug delivery composition (i.e., all of the discrete solid dosage forms together) comprise a total of about 200 mg to about 500 mg of the rasagiline hemitartrate. For example, the discrete solid dosage form(s) may comprise between about 300 mg to about 450 mg rasagiline hemitartrate, or about 350 mg to about 400 mg rasagiline hemitartrate.

The discrete solid dosage forms may be of any suitable shape and of any suitable quantity. In one embodiment of the present invention, the discrete solid dosage forms are cylindrical in shape. In another embodiment of the present invention, the discrete solid dosage forms are substantially spherical in shape. The discrete solid dosage form(s) may be "substantially spherical" in that the solid dosage forms are spherical or nearly spherical in that the length of the longest radius is approximately equal to the shortest radius of the dosage form. For example, the shape of the dosage form may not deviate from a perfect sphere by more than about 10%.

Without wishing to be bound by any theory, it is believed that the surface area of the at least one discrete dosage forms contributes to the elution rate. In one embodiment, the total surface area of the at least one discrete dosage forms is directly proportional to elution rate. Thus, the number of discrete dosage forms may be selected to provide a given elution rate, wherein an increased number of dosage forms provides an increased total surface area. The discrete solid dosage forms may comprise more than one pellet (*e.g.,* 2-12 pellets). In other words, a higher number of dosage forms may result in a higher average elution rate than a smaller number of dosage forms. Thus, it may be preferable to include more discrete solid dosage forms to give a higher elution rate (*e.g.,* 7-12 pellets). In a further embodiment, the overall surface area of the pellets used in the implantable drug delivery composition can be increased, for example by changing the shape of the pellets, increasing their surface convolution, etc.

The number of discrete solid dosage forms (e.g., pellets) may vary depending on the amount of rasagiline hemitartrate included in each solid dosage form. For example, each pellet may comprise between about 20 mg to about 100 mg rasagiline hemitartrate, for example about 60 mg rasagiline hemitartrate, or between about 30 mg to about 55 mg rasagiline hemitartrate, or between about 40 mg to about 50 mg rasagiline hemitartrate. According to one example, the drug delivery composition comprises about 9 pellets, with each pellet comprising between about 40 mg to about 45 mg rasagiline hemitartrate. See, e.g., Example 4 below, in which about 298 mg and about 385 mg of the rasagiline hemitartrate blend (i.e., each with 10% croscarmellose and 2 % stearic acid) were loaded into each implant by placing 7 pellets and 9 pellets into each implant, respectively.

The discrete solid dosage form(s) comprise the hemitartrate salt of rasagiline, and optionally, other active pharmaceutical ingredient(s). Rasagiline, which is a selective inhibitor of monoamine oxidase (MAO)-B, is also known as R-(+)-N-propargyl-1-aminoindan and has the following general formula:

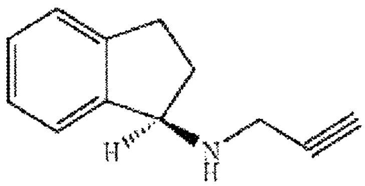

Reference herein to the delivery, release, or elution of rasagiline from an implant may include the delivery, release, or elution of rasagiline free base, active metabolites of rasagiline (*e.g.,* 1(*R*)-aminoindan), and/or rasagiline hemitartrate. The amount of rasagiline hemitartrate in compositions of the present invention is not particularly limited, but may be preferably on the order of about 75-97 wt% of the solid dosage form or 85-95 wt% of the solid dosage form (*e.g.,* about 88 wt%). The discrete solid dosage form may optionally include at least one other active pharmaceutical ingredient(s), such as levodopa.

The discrete solid dosage form may also comprise a sorption enhancer. As used herein, the term "sorption enhancer" is intended to encompass compounds which improve release of the API from the drug delivery composition. Without wishing to be bound to a particular theory, the sorption enhancers may improve release of the API from the drug delivery composition by drawing water or other fluids into the reservoir from the subject, disintegrating or breaking apart the discrete solid dosage form(s), and/or allowing the API to come into contact or remain in contact the inner walls of the excipient. Such a mechanism may be depicted, for example, in Figure 1. Figure 1 represents the rate-controlling excipient 110. The API, located in the reservoir on the left side of the diagram, is sorbed 112 from the reservoir to the excipient. The API then crosses through the excipient 110. The API is then desorbed 114 from the excipient into the subject.

Any suitable sorption enhancer(s) may be selected by one of ordinary skill in the art. Particularly suitable sorption enhancer(s) may include, for example, negatively-charged polymers, such as croscarmellose sodium, sodium carboxymethyl starch, sodium starch glycolate, sodium acrylic acid derivatives (e.g., sodium polyacrylate), cross-linked polyacrylic acid (e.g., CARBOPOL®), chondroitin sulfate, poly-glutamic acid, poly-aspartic acid, sodium carboxymethyl cellulose, neutral polymers, such as polyethylene glycol, polyethylene oxide, polyvinylpyrrolidone, and combinations thereof. In an exemplary embodiment, the sorption enhancer is croscarmellose sodium. The amount of the sorption enhancer may be present on the order of about 1-25 wt% of the solid dosage form, about 2-20 wt% of the solid dosage form, about 2-12 wt% of the solid dosage form, about 5-10 wt% of the solid dosage form (*e.g.,* about 5 wt% or about 10 wt% of the solid dosage form).

The amount of sorption enhancer may be proportional to the elution rate. In other words, a higher weight percent

of sorption enhancer in the drug composition may result in a higher average elution rate than a smaller weight percentage. Thus, it may be preferable to include a higher weight percent of sorption enhancer to give a higher elution rate (*e.g.,* 8-25 wt%).

The discrete solid dosage form may also comprise other ingredients as long as they do not adversely impact the elution rate. Other suitable ingredients may include, for example, lubricants, excipients, preservatives, etc. A lubricant may be used in the pelleting or tableting process to form the discrete solid dosage form(s), as would be well known by one of ordinary skill in the art. Suitable lubricants may include, but are not limited to, magnesium stearate, calcium stearate, zinc stearate, stearic acid, polyethylene glycol, and the like. The amount of any additional ingredients is not particularly limited, but is preferably on the order of less than about 5 wt% of the solid dosage form, and most preferably less than about 3 wt% of the solid dosage form, particularly preferably about 2% or less of the solid dosage form.

In one embodiment of the present invention, the at least one discrete solid dosage form comprises, consists essentially of, or consists of: 75-97 wt% rasagiline hemitartrate based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. For example, the at least one discrete solid dosage form comprises, consists essentially of, or consists of: 85-95 wt% (*e.g.,* 88 wt%) rasagiline hemitartrate based on the total weight of the at least one discrete solid dosage form; 5-20 wt% (*e.g.,* 10 wt%) of croscarmellose sodium based on the total weight of the at least one discrete solid dosage form; and 0-5 wt% (*e.g.*, 2 wt%) stearic acid based on the total weight of the at least one discrete solid dosage form. Preferably, each component of the drug delivery composition is provided in an amount effective for the treatment of one or more symptoms of Parkinson's disease.

Excipient

The discrete solid dosage form(s) is/are surrounded by an excipient. In other words, the discrete solid dosage form(s) is/are substantially or completely surrounded, encased, or enclosed by the excipient. In the present invention, there are no holes or pores in the excipient to allow egress of the API or ingress of bodily fluids, unlike an osmotic system, which requires a hole to allow release of the API. Moreover, there is no (or negligible) build up of pressure within a drug delivery composition in accordance with the present invention, unlike an osmotic system, which requires pressure to force the API out of the device.

In one embodiment of the present invention, the excipient is substantially or completely non-porous. "Substantially nonporous" may refer to a material which has a porosity or void percentage less than about 10%, about 5%, or about 1%, for example. In particular, the excipient is substantially non-porous in that there are no physical pores or macropores, which would allow for egress of the API from the drug delivery composition. In another embodiment, the excipient is practically insoluble in water. Solubility is the concentration of a solute when the solvent has dissolved all the solute that it can at a given temperature (*e.g.,* the concentration of solute in a saturated solution at equilibrium). As used herein, the term "practically insoluble in water" is consistent with the definition in The United States Pharmacopeia - National Formulary (USP-NF) definition, which provides for more than 10,000 parts solvent to one part solute (*e.g.,* one gram of the excipient in greater than 10,000 mL of water).

Without wishing to be bound to a particular theory, it is believed that a concentration gradient across the excipient (*e.g.,* wall, membrane, layer) allows for continuous release of the API. As depicted in Figure 1, sorption 112 of the API occurs from the reservoir onto the rate-controlling excipient 110. The API then dissolves into and fully saturates the excipient 110, diffuses through it, and the API is then desorbed 114 from the excipient into the subject. Accordingly, this gradient allows the "infinite" supply of API to be adsorbed onto the excipient, diffuse through it and desorbed into the subject, which, based on the excipient selected, may help to provide the pseudo-zero order release of the drug. Thus, the excipient may also be called a drug elution rate-controlling or rate-controlling excipient herein. The "rate-controlling excipient" is intended to encompass materials which control the elution rate of the API. In other words, a polymeric excipient, that when encasing the drug delivery composition, provides a different rate of release, namely, a controlled rate of release (*e.g.,* pseudo-zero order) as compared to the release of an API from an identical composition without a rate-controlling excipient.

The excipient defines the shape of the reservoir. The reservoir may be of any suitable size and shape. In an exemplary embodiment, the excipient is substantially cylindrically shaped. As used herein, the terms "cylindrical" or "cylindrically shaped" may be used interchangeably to mean at least substantially having the shape of a cylinder. As used herein, the term "cylinder" includes and refers to, but is not limited to: circular cylinders, having a circular cross-section; elliptical cylinders, having an elliptical cross-section; generalized cylinders, having any shape in cross-section; oblique cylinders, in which the end surfaces are not parallel to one another and/or are not normal to the axis of the cylinder; and conical and frusto-conical analogs thereof. In accordance with one aspect of the invention, a hollow tube may include a substantially consistent cross-sectional area and two substantially equally-sized circular ends. The cylindrical shape defines the shape of the excipient defining the reservoir (*e.g.,* the outer portion of the drug delivery composition). An embodiment of the cylindrically shaped excipient is depicted, for example, in Figure 2. Preferably, the dimensions of the cylindrical hollow tube should be as precise as possible (*e.g.*, a consistent shape and dimension along the length of the tube, in particular, a consistent circular cross-section). The reservoir may be of any suitable size depending on the active and location of delivery. For example, the composition may range in size from about 2mm to about 4mm in diameter (*e.g.,* about 2.7 mm in diameter) and about 6mm to about 50mm in length, for example about 45 mm in length.

The excipient comprises at least one polymer. Any suitable polymer may be selected by one of ordinary skill in the art, as long as the polymer allows for delivery of a therapeutically effective amount of the API to the subject, for example, at a pseudo-zero order rate, for the intended period of time that the implant resides in a patient. In one embodiment, the polymer comprises a thermoplastic elastomer. As used herein, "thermoplastic," "thermoplastic elastomers (TPE)" or "thermoplastic rubbers" may be used to denote a class of copolymers or a physical mix of polymers (*e.g.,* a plastic and a rubber), which consist of materials with both thermoplastic and elastomeric properties. The crosslinking in thermoplastic elastomeric polymers may include a weaker dipole or hydrogen bond or the crosslinking occurs in one of the phases of the material. The class of copolymer may include, for example, styrenic block copolymers, polyolefin blends, elastomeric alloys, thermoplastic polyurethanes, thermoplastic copolyester, and thermoplastic polyamides.

As used herein, "elastomer" or "elastomeric polymer" is intended to encompass polymers (homopolymers, copolymers, terpolymers, oligomers, and mixtures thereof) having elastomeric properties (*e.g.,* the tendency to revert to its original shape after extension). In other words, the polymeric backbone may contain one or more elastomeric subunits (*e.g*., an elastomeric soft segment or block). In one embodiment, the elastomeric polymer comprises polyurethane, polyether, polyamide, polycarbonate, polysilicone, or copolymers thereof. Thus, the elastomeric polymer may include polyurethane-based polymers, polyether-based polymers, polysilicone-based polymers, polycarbonate-based polymers, or combinations thereof.

The polymer may be formed by any suitable means or techniques known to one of ordinary skill in the art. For example, the polymer may be formed from monomers, polymer precursors, pre-polymers, polymers, etc. Polymer precursors may include monomeric as well as oligomeric substances capable of being reacted or cured to form polymers. The polymers may be synthesized using any suitable constituents.

In one embodiment of the present invention, the polymer comprises polyurethanes (*e.g.,* comprising a urethane linkage, -RNHCOOR'-). Polyurethanes may include polyether-based polyurethanes, polycarbonate-based polyurethanes, polyamide-based polyurethanes, polysilicone-based polyurethanes, or the like. Polyurethanes may be formed, for example, from polyols (*e.g*., comprising two or more hydroxyl or alcohol functional groups, -OH), isocyanates (*e.g*., comprising an isocyanate group, -N=C=O), and, optional chain extenders, catalysts, and other additives.

Suitable polyols may include, for example, polyether polyols, polycarbonate-based polyols, and the like, which may include diols, triols, etc. Polyether polyols may include, for example, polyalkylene glycols (*e.g.,* polyethylene glycols, polypropylene glycols, polybutylene glycols), poly(ethylene oxide) polyols (*e.g.,* polyoxyethylene diols and triols), polyoxypropylene diols and triols, and the like. Alternative polyols may include, for example, 1,4-butanediol, 1,6-hexanediol, 1,12-dodecanediol, and the like.

For example, the polyol segment or segments may be represented by one or more of the following formulas:

$$O\text{-}(CH_2\text{-}CH_2\text{-}CH_2\text{-}CH_2)_x\text{-}O\text{-} \qquad \text{(Formula 1)}$$

$$\text{-}[O\text{-}(CH_2)_n]_x\text{-}O\text{-} \qquad \text{(Formula 2)}$$

$$O\text{-}[(CH_2)6\text{-}CO_3]_n\text{-}(CH_2)\text{-}O\text{-} \qquad \text{(Formula 3)}$$

Formula (1) may depict a suitable polyether-based polyol, which may be representative of a polyol to produce TECOFLEX® polyurethanes. Formula (2) may depict a suitable polyether-based polyol, which may representative of a polyol to produce TECOPHILIC® polyurethanes. Formula (3) may depict a suitable polycarbonate-based polyol, which may be representative of a polyol to produce CARBOTHANE® polyurethanes (all of which are obtainable from the Lubrizol Corporation with offices in Wickliffe, Ohio). The polyols may also include mixtures of one or more types of polyol segments.

Suitable isocyanates may include, for example, aliphatic and cycloaliphatic isocyanates, as well as aromatic isocyanates, such as 1,6-hexamethylene diisocyanate (HDI), 1-isocyanato-3-isocyanatomethyl-3,5,5-trimethyl-cyclohexane (isophorone diisocyanate, IPDI), and 4,4'-diisocyanato dicyclohexylmethane (H12MDI), as well as methylene diphenyl diisocyanate (MDI) and toluene diisocyanate (TDI).

Suitable chain extenders may include, for example, ethylene glycol, 1,4-butanediol (1,4-BDO or BDO), 1,6-hexanediol, cyclohexane dimethanol, and hydroquinone bis(2-hydroxyethyl) ether (HQEE).

In one embodiment of the present invention, the polymer comprises a polyether-based polyurethane. For example, the polymer may be an aliphatic polyether-based polyurethane comprising poly(tetramethylene oxide) and

polymerized 4,4'-diisocyanato dicyclohexylmethane (H12MDI) and 1,4-butanediol. An exemplary type of suitable polyether-based polyurethanes includes TECOFLEX® polymers available from the Lubrizol Corporation. For example, TECOFLEX® polymers include aliphatic block copolymer with a hard segment consisting of polymerized 4,4'-diisocyanato dicyclohexylmethane (H12MDI) and 1,4-butanediol, and a soft segment consisting of the macrodiol poly(tetramethylene oxide). In one embodiment, the TECOFLEX® polymer comprises TECOFLEX® EG-93A polyurethane. In another embodiment, the TECOFLEX® polymer comprises TECOFLEX® EG-80A polyurethane.

In another embodiment of the present invention, the polymer comprises polyether-amides (*e,g*., thermoplastic poly(ether-block-amide)s, *e,g*., PEBA, PEB, TPE-A, and commercially known as PEBAX® polyether-amides obtainable from Arkema Chemicals Inc., headquartered in King of Prussia, PA). Synthesis may be carried out, for example, in the molten state by polycondensation between polyether blocks (*e.g.,* a diol, such as polyoxyalkylene glycols) and polyamide blocks (*e.g*., carboxylic acid terminated amide blocks, such as dicarboxylic blocks), which results in a thermoplastic copolymer. The long chain molecules may consist of numerous blocks where the polyamide provides rigidity and the polyether provides flexibility to the polymer. Thus, the polyether-amides may consist of linear chains of hard polyamide (PA) blocks covalently linked to soft polyether (PE) blocks via ester groups. The polyether-amides may also be synthesized via a catalyst (*e.g*., metallic $Ti(OR)_4$), which facilitates the melt polycondensation of the polyether and polyamide blocks. The general structural formula of these block copolymers may be depicted as follows:

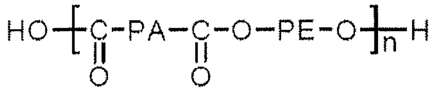

(Formula 4)

The polyamide block may include various amides including nylons (such as nylon 6, nylon 11, nylon 12, etc.). The polyether block may also include various polyethers, such as polytetramethylene oxide (PTMO), polypropylene oxide (PPO), polyethylene glycol (PEG), poly(hexamethylene oxide), polyethylene oxide (PEO), and the like. The ratio of polyether to polyamide blocks may vary from 80:20 to 20:80 (PE:PA). As the amount of polyether increases, a more flexible, softer material may result.

For example, the thermoplastic elastomer may be selected from the group consisting of TECOFLEX® polyurethanes, CARBOTHANE® polyurethanes, PEBAX® polyether-amides, and combinations thereof. For example, the elastomer may include TECOFLEX® EG-93A polyurethane, TECOFLEX® EG-80A polyurethane, TECOFLEX® EG-85A polyurethane, PEBAX® 2533 polyether-amide, PEBAX® 3533 polyether-amide, CARBOTHANE® PC-3585A polyurethane, and combinations thereof.

TECOFLEX® polyurethanes and CARBOTHANE® polyurethanes are described, for example, in Lubrizol's brochure for Engineered Polymers for Medical & Healthcare dated 2011, the disclosure of which is hereby incorporated by reference in its entirety, for all purposes. For example, TECOFLEX® aliphatic polyether polyurethanes may have the following characteristics:

**Table 1**

| Product | Hardness | Flex Modulus | Feature |
|---|---|---|---|
| EG80A | 72A | 1,000 | Clear |
| EG85A | 77A | 2,300 | Clear |
| EG93A | 87A | 3,200 | Clear |
| EG100A | 94A | 10,000 | Clear |
| EG60D | 51D | 13,000 | Clear |
| EG65D | 60D | 37,000 | Clear |
| EG68D | 63D | 46,000 | Clear |
| EG72D | 67D | 92,000 | Clear |
| EG80A B20/B40 | 73A/78A | 1,200/1,500 | Radiopaque |
| EG85A B20/B40 | 83A/86A | 2,700/3,700 | Radiopaque |
| EG93A B20/B40 | 90A/95A | 5,000/4,700 | Radiopaque |
| EG100A B20/B40 | 93A/98A | 17,000/14,000 | Radiopaque |

(continued)

| Product | Hardness | Flex Modulus | Feature |
|---|---|---|---|
| EG60D B20/B40 | 55D/65D | 27,000/27,000 | Radiopaque |
| EG65D B20/B40 | 63D/78D | 82,000/97,000 | Radiopaque |
| EG68D B20 | 73D | 76,600 | Radiopaque |
| EG72D B20/B40 | 75D/82D | 125,000/179,000 | Radiopaque |

CARBOTHANE® aliphatic polycarbonate polyurethanes may have the following characteristics, for example:

**Table 2**

| Product | Hardness | Flex Modulus | Feature |
|---|---|---|---|
| PC-3575A | 71A | 620 | Clear |
| PC-3585A | 78A | 1,500 | Clear |
| PC-3595A | 91A | 4,500 | Clear |
| PC-3555D | 52D | 24,000 | Clear |
| PC-3572D | 71D | 92,000 | Clear |
| PC-3575A-B20 | 79A | 860 | Radiopaque |
| PC-3585A-B20 | 81A | 1,700 | Radiopaque |
| PC-3595A-B20 | 90A | 8,600 | Radiopaque |
| PC-3555D-B20 | 54D | 25,000 | Radiopaque |
| PC-3572D-B20 | TBD | 141,000 | Radiopaque |

The polymers may be processed using any suitable techniques, such as extrusion, injection molding, compression molding, spin-casting. For example, the polymer may be extruded or injection molded to produce hollow tubes having two open ends (see *e.g.,* Figure 2). The hollow tube can be loaded with the discrete solid dosage form(s). The open ends are sealed to form the reservoir-based drug delivery composition. A first open end may be sealed before filling the tube with the discrete solid dosage form(s), and the second open end may be sealed after the tube is filled with all of the discrete solid dosage form(s). The tube may be sealed using any suitable means or techniques known in the art. For example, the ends may be plugged, filled with additional polymers, heat sealed, or the like. The tubes should be permanently sealed such that the discrete solid dosage form(s) may not be removed. Also, the ends should be suitably sealed such that there are no holes or openings that would allow egress of the active once implanted.

The wall thickness of the excipient may be selected to provide for the desired elution rate. The wall thickness may be inversely proportional to elution rate. Thus, a larger wall thickness may result in a lower elution rate. The excipient may form a wall having an average thickness of about 0.05 to about 0.5 mm, or about 0.1 mm to about 0.3 mm (*e.g.*, about 0.1 mm, about 0.2 mm, or about 0.3 mm).

In one embodiment of the present invention, the drug delivery composition does not require erosion or degradation of the excipient *in vivo* in order to release the API in a therapeutically effective amount. Alternatively, the excipient is not substantially erodible and/or not substantially degradable *in vivo* for the intended life of the implantable composition. As used herein, "erosion" or "erodible" are used interchangeably to mean capable of being degraded, disassembled, and/or digested, *e.g.,* by action of a biological environment. A compound that is "not substantially erodible" is not substantially degraded, disassembled, and/or digested over time (*e.g.,* for the life of the implant). Alternatively, the material may be "not substantially erodible" or "does not require erosion" *in vivo* in order to provide for release of the API. In other words, the compound may erode over time, but the API is not substantially released due to erosion of the material. With respect to "degradation" or "degradable," these are intended to mean capable of partially or completely dissolving or decomposing, *e.g.,* in living tissue, such as human tissue. Degradable compounds can be degraded by any mechanism, such as hydrolysis, catalysis, and enzymatic action. Accordingly, a compound that is "not substantially degradable" does not substantially dissolve or decompose over time (*e.g.,* for the life of the implant) *in vivo.* Alternatively, the material may be "not substantially degradable" or "not requiring degradation" in order to provide for release of the API. In other words, the compound may degrade over time, but the API is not substantially released due to degradation of the material.

Implantation

The method of treating one or more symptoms of Parksinson's disease includes implanting a reservoir-based drug delivery composition into a subject. The term "subject" or "patient", used herein, refers to a mammalian subject, such as a human being. The subject is preferably a human that has been diagnosed with Parkinson's disease (*e.g.,* the subject has either early or advanced Parkinson's disease) and/or exhibits one or more symptoms of Parkinson's disease.

The drug delivery composition may be implanted into the subject in any suitable area of the subject using any suitable means and techniques known to one of ordinary skill in the art. For example, the composition may be implanted subcutaneously, *e.g.,* at the back of the upper arm or the upper back (*e.g.* in the scapular region). As used herein, the terms "subcutaneous" or "subcutaneously" or "subcutaneous delivery" means directly depositing in or underneath the skin, a subcutaneous fat layer, or intramuscularly. The drug delivery composition may be delivered subcutaneously using any suitable equipment or techniques. In one embodiment, the drug delivery composition is placed subcutaneously in the subject's arm. Alternative sites of subcutaneous administration may also be used as long as a pharmaceutically acceptable amount of the API would be released into the subject in accordance with the present invention. Preferably, the drug delivery composition should not migrate significantly from the site of implantation. Methods for implanting or otherwise positioning the compositions into the body are well known in the art. Removal and/or replacement may also be accomplished using suitable tools and methods known in the art.

Once implanted, the reservoir-based drug delivery composition may systemically deliver a therapeutically effective amount of the rasagiline to the subject at a pseudo-zero order rate (e.g., zero order rate) for a long duration (*e.g.,* a period of time of at least one month). As used herein, the term "systemic" or "systemically" refers to the introduction of the API into the circulatory, vascular and/or lymphatic system (*e.g*,, the entire body). This is in contrast to a localized treatment where the treatment would only be provided to a specific, limited, localized area within the body. Thus, the API is systemically delivered to the subject by implanting the drug delivery composition subcutaneously into the subject.

A therapeutically effective amount of the rasagiline is preferably delivered to the subject at a pseudo-zero order rate. Pseudo-zero order refers to a zero-order, near-zero order, substantially zero order, or controlled or sustained release of the rasagiline. A pseudo-zero order release profile may be characterized by approximating a zero-order release by release of a relatively constant amount of the rasagiline per unit time (*e.g.,* within about 30% of the average value). Thus, the composition may initially release an amount of the rasagiline that produces the desired therapeutic effect, and gradually and continually release other amounts of the rasagiline to maintain the level of therapeutic effect over the intended duration (*e.g.,* about one year). In order to maintain a near-constant level of rasagiline in the body, the rasagiline may be released from the composition at a rate that will replace the amount of rasagiline being metabolized and/or excreted from the body.

Without wishing to be bound to a particular theory, it is believed that the reservoir-based drug composition works by releasing the active (*e.g.,* rasagiline) through the excipient membrane or wall. In other words, the rasagiline diffuses across the excipient, *e.g.,* as depicted in Figure 1. Thus, sorption 112 of the rasagiline occurs from the reservoir onto the rate-controlling excipient 110. The rasagiline fully saturates the excipient 110 at steady state, and the rasagiline diffuses through the excipient and is then desorbed 114 from the excipient into the subject at a pseudo-zero order rate.

The therapeutically effective amount of the rasagiline may be delivered to the subject at a target range between a maximum value and a minimum value of average daily elution rate for the API. As used herein, the term "elution rate" refers to a rate of API delivery, which is based on the oral dose rate multiplied by the fractional oral bioavailability, which may be depicted as follows:

$$\text{Oral Dose X Fractional Oral Bioavailability \%} = \text{Target Elution Rate (mg/day)}$$

The elution rate may be an average rate, *e.g.,* based on the mean average for a given period of time, such as a day (i.e., average daily elution rate). Thus, a daily elution rate or average daily elution rate may be expressed as target daily oral dosage multiplied by oral bioavailability. For example, in the case of the oral dosage form of rasagiline mesylate, which has an approximate oral bioavailability of 35% and a target oral daily dose of 0.5 mg/day to 1 mg/day, a target daily elution rate for rasagiline is about 175 micrograms per day to about 350 micrograms per day. Other elution rates are contemplated; for example, an approximate minimum elution rate of 100 micrograms per day, and an approximate maximum elution rate of 10,000 micrograms per day.

The maximum and minimum values refer to a maximum average daily elution rate and a minimum average daily elution rate, respectively. The minimum value required for a pharmaceutically effective dose may be correlated to or determined from a trough value for an oral dosage version of the API (*e.g.*, based on the blood/plasma concentrations for oral formulations). Similarly, maximum value may be correlated to or determined from the peak value for an oral dosage version of the API (*e.g*,, the maximum blood/plasma concentration when an oral dosage is first administered or

a pharmaceutically toxic amount). In other words, the target range is a range between maximum and minimum average daily elution rates, respectively, which may be determined based on blood/plasma concentrations for equivalent oral dosage forms containing the same active..

In one embodiment of the present invention, rasagiline is delivered to the subject at a target range of about 100 micrograms/day to about 1000 micrograms/day. For example, rasagiline is delivered to the subject at a target range of about 200 to about 600 micrograms/day, or about 250 to about 500 micrograms per day, or about 300 to about 400 micrograms per day, or about 175 to about 350 micrograms per day or about 700 micrograms/day. In preferred embodiments, rasagiline is delivered to the subject at an average rate of about 350 micrograms per day. The testing method set forth in the examples to determine the elution rates for compositions comprising rasagiline includes placing the implants in an elution bath consisting of 50 mL 0.9% saline at 37°C. Weekly exchanges of the elution media are then analyzed by HPLC for the durations given.

The drug delivery compositions of the present invention are long-lasting. In other words, rasagiline is delivered to the subject (*e.g.,* at a pseudo-zero order rate) for an extended period of time. For example, rasagiline is delivered to the subject for at least about one month (about one month or greater), at least about three months (about three months or greater), at least about six months (about six months or greater), at least about one year (about one year or greater), at least about 18 months (about 18 months or greater), at least about two years (about two years or greater), at least about 30 months (about 30 months or greater), or any period of time within those ranges. Figures 4 and 6, for example, show *in vitro* elution rates of rasagiline at a pseudo-zero order rate over 21 weeks and 31 weeks, respectively. Figure 7 shows *in vivo* plasma concentrations (ng/mL) of rasagiline over about 16 weeks.

According to one embodiment, a method of treating one or more symptoms of Parkinson's disease comprises implanting a reservoir-based drug delivery composition into a subject to systemically deliver a therapeutically effective amount of rasagiline to the subject for a period of time of at least about one year (e.g., about one year), wherein the drug delivery composition comprises at least one discrete solid dosage form comprising rasagiline hemitartrate surrounded by an excipient comprising at least one polymer, at an average daily elution rate of about 100 to about 1000 micrograms/day (e.g., about 700 micrograms/day or about 350 micrograms/day), wherein the at least one discrete solid dosage form comprises, consists essentially of, or consists of 75-97 wt% rasagiline hemitartrate (e.g., about 88% rasagiline hemitartrate), 1-25 wt% of at least one sorption enhancer (e.g., about 10% croscarmellose sodium), and 0-5 wt% lubricant (e.g., about 2% stearic acid), all based on the total weight of the at least one discrete solid dosage form. The at least one discrete solid dosage form may comprise between about 200 to about 500 mg of the rasagiline hemitartrate (e.g., about 275 mg to about 450 mg, or about 300 mg to about 400 mg, or about 385 mg).

According to another embodiment, a method of treating one or more symptoms of Parkinson's disease comprises implanting a reservoir-based drug delivery composition into a subject to systemically deliver a therapeutically effective amount of rasagiline to the subject for a period of time of about two years or more (e.g., about two years or about 30 months), wherein the drug delivery composition comprises at least one discrete solid dosage form comprising rasagiline hemitartrate surrounded by an excipient comprising at least one polymer, at an average daily elution rate of about 100 to about 1000 micrograms/day (e.g., about 700 micrograms/day or about 350 micrograms/day), wherein the at least one discrete solid dosage form comprises, consists essentially of, or consists of 75-97 wt% rasagiline hemitartrate (e.g., about 90% rasagiline hemitartrate), 1-25 wt% of at least one sorption enhancer (e.g., about 9% croscarmellose sodium), and 0-5 wt% lubricant (e.g., about 1% stearic acid), all based on the total weight of the at least one discrete solid dosage form. The at least one discrete solid dosage form may comprise between about 200 to about 500 mg of the rasagiline hemitartrate (e.g., about 450 mg).

Prior to implantation, the drug delivery composition may undergo any suitable processing, such as sterilization (such as by gamma radiation), heat treatment, molding, and the like. Additionally, the drug delivery composition may be conditioned or primed by techniques known in the art. For example, the drug delivery composition may be placed in a medium (*e.g.,* an aqueous medium, such as saline). The medium, priming temperature, and time period of priming can be controlled to optimize release of the active upon implantation.

## Efficacy of Treatment for Parkinson's disease

The methods of treatment described herein may treat, delay onset, suppress, or inhibit one or more symptoms of Parkinson's disease. A pharmaceutically effective or therapeutic amount of rasagiline should be administered sufficient to effect or produce the desired therapy. For example, releasing an amount of rasagiline effective to inhibit or suppress one or more symptoms of Parkinson's disease (e.g., bradykinesia, tremors, muscular rigidity, and/or postural instability) is desired. A doctor would be able to determine the efficacy of the treatment (i.e., know the rasagiline was working to treat symptoms of Parkinson's disease) using techniques known to one of ordinary skill in the art.

For example, after a subject has begun a regimen of rasagiline, a clinician may use a rating scale which assesses the symptoms of Parkinson's disease in order to determine whether there has been an improvement in those symptoms over time. One measure of effectiveness is the Unified Parkinson's Disease Rating Scale (UPDRS). The UPDRS is a

widely-used scale with four sections. Part I assesses mentation, behavior, and mood (e.g., intellectual impairment). Part II assesses activities of daily living (e.g., speech, handwriting, use of utensils, falling, dressing, walking, etc.). Part III is the motor examination (e.g., speech, facial expression, tremors at rest, rigidity, postural stability, bradykinesia, etc.). Part IV assesses complications of therapy. The total scale comprises 199 points, with the motor examination accounting for 108 points. A reduction in the score represents improvement and a beneficial change from baseline appears as a negative number.

Improvement in a subject's symptoms, as measured by a clinician according to the aforementioned assessment, or other assessments used in the art to evaluate the symptoms of Parkinson's disease, can be used to indicate whether the amount of rasagiline being used is effective. For example, the effectiveness of rasagiline in treating a subject's symptom(s) of Parkinson's disease may comprise an improvement of at least about 10%, at least about 20%, or at least about 30% in the patient's UPDRS score over a period of time (e.g., about 1 month, about 3 months, about six months, or about one year) following the start of a rasagiline regimen (e.g., following implantation).

It would also be appreciated by one of ordinary skill in the art that the treatment regime for treating one or symptoms of Parkinson's disease with rasagiline may depend on a variety of factors, including the type, age, weight, sex, diet and medical condition of the subject. Thus, the treatment regime actually employed may vary widely from subject to subject.

Subcutaneous Delivery Systems and Kits

In one aspect of the present invention, a subcutaneous delivery system comprises an elastomeric reservoir implant comprising at least one discrete solid dosage form surrounded by a polymeric rate-controlling excipient. The at least one discrete solid dosage form comprises rasagiline hemitartrate. The subcutaneous delivery system provides for release of the rasagiline at an elution rate suitable to provide a therapeutically effective amount of the rasagiline to a subject at a pseudo-zero order rate for a period of time of at least one month. In another aspect of the present invention, a kit for subcutaneously placing a drug delivery composition comprises a reservoir-based drug delivery composition comprising a polymeric rate-controlling excipient defining a reservoir containing at least one discrete solid dosage form comprising rasagiline hemitartrate; and an implanter for inserting the reservoir-based drug delivery composition beneath the skin.

The drug delivery composition may be implanted into the subject in any suitable area of the subject using any suitable means and techniques known to one of ordinary skill in the art. For example, the composition may be implanted subcutaneously, e.g., at the back of the upper arm, by directly depositing in or underneath the skin, a subcutaneous fat layer, or intramuscularly.

The drug delivery composition may be delivered subcutaneously using any suitable equipment or techniques, *e.g.*, an implanter known to one ordinary skill in the art. The kits may comprise the drug delivery composition pre-loaded into the implanter or the drug delivery composition may be loaded by the doctor or other user. The implanter may be an implantation device, such as a syringe, cannula, trocar or catheter, that may be inserted into an incision made at the delivery site of the subject. Suitable implantation devices and implantation methods include the trocar and methods disclosed in US 7,214,206 and US 7,510,549, the disclosures of which are herein incorporated by reference in their entirety, for all purposes. Other suitable methods for implanting or otherwise positioning the compositions into the body, e.g., by a doctor, are well known in the art. Removal and/or replacement may also be accomplished using suitable tools and methods known in the art. Kits may also comprise other equipment well known in the art, such as scalpels, clamps, suturing tools, hydration fluid, and the like.

Implantable Drug Delivery Compositions with Polymer Excipient(s)

Without wishing to be bound to a particular theory, it is believed that by selecting specific polymers with certain contents or ratios of hard to soft segments, certain desired elution rates may be achieved. Moreover, by adding certain sorption enhancers in certain amounts with the rasagiline to the discrete solid dosage formulations within the reservoir, the elution rates may be further changed or modulated (*e.g.*, "tuned" or "dialed in") from the drug delivery composition to desired, pharmaceutically efficacious values.

According to one aspect of the present invention, a method of delivering a therapeutically effective amount of rasagiline from an implantable drug delivery composition comprises implanting a reservoir-based drug delivery composition into a subject to systemically deliver a therapeutically effective amount of rasagiline to the subject at a pseudo-zero order rate (e.g., zero order rate) for a period of time of at least one month. The drug delivery composition comprises at least one discrete solid dosage form surrounded by an excipient comprising at least one polymer, and the at least one discrete solid dosage form comprises rasagiline hemitartrate. The polymer comprises a substantially non-porous, elastomeric polymer comprising soft and hard segments, and the relative content of the soft and hard segments provide an elution rate within a target range between a maximum and minimum value of a desired average daily elution rate for

the rasagiline.

According to one embodiment of the present invention, a drug delivery composition includes a rate-controlling excipient defining a reservoir which contains at least one discrete solid dosage form comprising rasagiline hemitartrate. The rate-controlling excipient comprises a substantially non-porous, elastomeric polymer comprising soft and hard segments selected based on the relative content of soft and hard segments of the polymer to obtain an elution rate within a target range of average daily elution rate for the rasagiline. The at least one discrete solid dosage form comprises at least one sorption enhancer in an amount effective to modulate the average daily elution rate of the rasagiline to provide for release of the rasagiline at pseudo-zero order within the target range at the therapeutically effective amount for a period of time of at least one month. The amount of sorption enhancer may be directly proportional to the average daily elution rate.

According to another embodiment of the present invention, a method of choosing an implantable drug delivery composition comprises selecting a rate-controlling excipient comprising a substantially non-porous, elastomeric polymer comprising soft and hard segments for defining a reservoir based on the relative content of soft and hard segments of the polymer to adjust the elution rate within a target range of average daily elution rate for rasagiline; and selecting and formulating the rasagiline hemitartrate and at least one sorption enhancer in order to modulate the elution rate to achieve a therapeutically effective amount of the rasagiline at pseudo-zero order for a period of time of at least one month, wherein the amount of sorption enhancer may be directly proportional to the average daily elution rate.

Polymer Selection

The excipient comprises at least one polymer having soft and hard segments. As used herein, the term "segment" may refer to any portion of the polymer including a monomer unit, or a block of the polymer, or a sequence of the polymer, etc. "Soft segments" may include a soft phase of the polymer, which is amorphous with a glass transition temperature below the use temperature (*e.g.*, rubbery). "Hard segments" may include a hard phase of the polymer that is crystalline at the use temperature or amorphous with a glass transition temperature above the use temperature (*e.g.,* glassy). The use temperature may include a range of temperatures including room temperature (about 20-25 °C) and body temperature (about 37 °C). Without wishing to be bound to a particular theory, the soft segment may provide for the greatest impact on sorption onto the excipient and the hard segment may impact diffusion across or through the excipient. See *e.g.,* Figure 1 showing sorption 112 of the API from the reservoir into the excipient 110 and desorption 114 of the API from the excipient into the subject. Any suitable polymer comprising hard and soft segments may be selected by one of ordinary skill in the art, as long as the polymer allows for delivery of a therapeutically effective amount of the API to the subject at a pseudo-zero order rate for the intended period of time of the implant. In one embodiment of the present invention, the selected polymer excipient is hydrophobic.

In one embodiment, the polymer is a thermoplastic elastomer or elastomeric polymer, which encompasses polymers (homopolymers, copolymers, terpolymers, oligomers, and mixtures thereof) having elastomeric properties and containing one or more elastomeric subunits (*e.g.,* an elastomeric soft segment or block). The thermoplastic elastomers may include copolymers (*e.g.,* styrenic block copolymers, polyolefin blends, elastomeric alloys, thermoplastic polyurethanes, thermoplastic copolyester, and thermoplastic polyamides) or a physical mix of polymers (*e.g.,* a plastic and a rubber), which consist of materials with both thermoplastic and elastomeric properties, for example, comprising a weaker dipole or hydrogen bond or crosslinking in one of the phases of the material. The elastomeric polymer may comprise polyurethanes, polyethers, polyamides, polycarbonates, polysilicones, or copolymers thereof. Thus, the polymer may include elastomeric polymers comprising polyurethane-based polymers, polyether-based polymers, polysilicone-based polymers, polycarbonate-based polymers, or combinations thereof. In an exemplary embodiment, the polymer comprises a polyurethane-based polymer or a polyether-block-polyamide polymer.

Suitable hard and soft segments of the polymer may be selected by one of ordinary skill in the art. It will be appreciated by one of ordinary skill in the art that although certain types of polymers are described herein, the hard and soft segments may be derived from monomers, polymers, portions of polymers, etc. In other words, the polymers listed may be changed or modified during polymerization, but those polymers or portions of those polymers in polymerized form constitute the hard and soft segments of the final polymer.

Examples of suitable soft segments include, but are not limited to, those derived from (poly)ethers, (poly)carbonates, (poly)silicones, or the like. For example, the soft segments may be derived from alkylene oxide polymers selected from the group consisting of poly(tetramethylene oxide) (PTMO), polyethylene glycol (PEG), poly(propylene oxide) (PPO), poly(hexamethylene oxide), and combinations thereof. The soft segment may also be derived from polycarbonate soft segments (obtainable from Lubrizol) or silicone soft segments (obtainable from Aortech).

Examples of suitable hard segments include, but are not limited to, those derived from polyurethanes or polyamides. For example, the hard segments may be derived from isocyanates and amides, such as nylons, nylon derivatives (such as nylon 6, nylon 11, nylon 12, etc.), carboxylic acid terminated amide blocks, and the like.

The polymer may be formed by any suitable means or techniques known to one of ordinary skill in the art. For

example, the polymer may be formed from monomers, polymer precursors, pre-polymers, polymers, etc. Polymer precursors may include monomeric as well as oligomeric substances capable of being reacted or cured to form polymers. The polymers may be synthesized using any suitable constituents.

In one embodiment of the present invention, the polymer comprises polyurethanes (*e.g.,* comprising a urethane linkage, -RNHCOOR'-). Polyurethanes may include polyether-based polyurethanes, polycarbonate-based polyurethanes, polyamide-based polyurethanes, polysilicone-based polyurethanes, or the like, as discussed in detail above.

Polyurethanes may contain both soft segments and hard segments. The soft segments may be derived from polyols including polyether polyols, polycarbonate-based polyols, and the like. For example, soft segments may be derived from polyether polyols, such as polyalkylene glycols (*e.g.*, polyethylene glycols, polypropylene glycols, polybutylene glycols, polyoxyethylene diols and triols), polyoxypropylene diols and triols, and the like. Soft segments may alternatively be derived from polyols, such as 1,4-butanediol, 1,6-hexanediol, 1,12-dodecanediol, and the like. An elution rate for a composition comprising a polycarbonate soft segment polyurethane is provided in Figure 12. The soft segment derived from the polyols may be represented by the following formulas or mixtures thereof, for example:

$$O\text{-}(CH_2\text{-}CH_2\text{-}CH_2\text{-}CH_2)_x\text{-}O\text{-} \qquad \text{(Formula 1)}$$

$$\text{-}[O\text{-}(CH_2)_n]_x\text{-}O\text{-} \qquad \text{(Formula 2)}$$

$$O\text{-}[(CH_2)_6\text{-}CO_3]_n\text{-}(CH_2)\text{-}O\text{-} \qquad \text{(Formula 3)}$$

The hard segments may be derived from isocyanates, such as aliphatic and cycloaliphatic isocyanates, as well as aromatic isocyanates, such as 1,6-hexamethylene diisocyanate (HDI), 1-isocyanato-3-isocyanatomethyl-3,5,5-trimethylcyclohexane (isophorone diisocyanate, IPDI), and 4,4'-diisocyanato dicyclohexylmethane (H12MDI), as well as methylene diphenyl diisocyanate (MDI) and toluene diisocyanate (TDI).

In another embodiment of the present invention, the polymer may comprise a polyether-based polyurethane. For example, the polymer may be an aliphatic polyether-based polyurethane comprising poly(tetramethylene oxide) as the soft segment and polymerized 4,4'-diisocyanato dicyclohexylmethane (H12MDI) and 1,4-butanediol as the hard segment. A suitable polymer includes a polymer from the TECOFLEX® family, an aliphatic block copolymer with a hard segment consisting of polymerized 4,4'-diisocyanato dicyclohexylmethane (H12MDI) and 1,4-butanediol, and a soft segment consisting of the macrodiol poly(tetramethylene oxide).

In another embodiment of the present invention, the polymer comprises polyether-amides *(e.g.,* thermoplastic poly(ether-block-amide)s, *e.g.,* PEBA, PEB, TPE-A, and commercially known as PEBAX® polyether-amides). The hard segment may comprise the polyamide blocks (*e.g.,* carboxylic acid terminated amide blocks, such as dicarboxylic blocks) and the soft segments may comprise the polyether blocks (*e.g.,* a diol, such as polyoxyalkylene glycols). The general structural formula of these block copolymers may be depicted as follows:

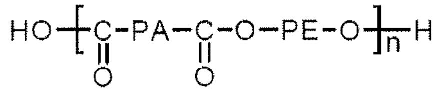

(Formula 4)

where PA represents the hard segment and PE represents the soft segment. The polyamide block may include various amides including nylons (such as nylon 6, nylon 11, nylon 12, etc.). The polyether block may also include various polyethers, such as poly(tetramethylene oxide) (PTMO), polyethylene glycol (PEG), poly(propylene oxide) (PPO), poly(hexamethylene oxide), polyethylene oxide (PEO), and the like. The ratio of polyether to polyamide blocks may vary from 80:20 to 20:80 (PE:PA). As the amount of polyether increases, a more flexible, softer material may result.

In one embodiment, the elastomeric polymer is selected from the group consisting of TECOFLEX® polyurethanes, CARBOTHANE® polyurethanes, PEBAX® polyether-amides, and combinations thereof. For example, the elastomeric polymer may include TECOFLEX® EG-93A polyurethane, TECOFLEX® EG-80A polyurethane, TECOFLEX® EG-85A polyurethane, PEBAX® 2533 polyether-amide, PEBAX® 3533 polyether-amide, CARBOTHANE® PC-3585A polyurethane, and combinations thereof.

The relative content of the soft and hard segments may provide an elution rate within a target range of average daily elution rate for the active pharmaceutical ingredient. The relative content of the soft and hard segments refers to the amount or content of soft segments to hard segments in the polymer. The relative content may also be defined as a ratio of soft segment to hard segments (*e.g.,* at least about 2:1 or at least about 4:1 of soft to hard segments). For example, the soft content may be 50% or more, 60% or more, 70% or more, or 80% or more relative to the hard content. In one embodiment, the relative content is about 70% soft segments and about 30% hard segments or at least about 2.3:1 soft:hard (e.g., PEBAX® 2533 polyether-amide). In another embodiment, the relative content is about 80% soft

segments and about 20% hard segments or at least about 4:1 soft:hard (e.g., PEBAX® 3533 polyether-amide).

The ratio of soft to hard segments may vary depending on the desired elution rate. Without wishing to be bound to a particular theory, it is believed that the soft segments may contribute to the sorption of the API into the excipient and/or the hard segment may contribute to the rate of diffusion (*e.g.*, how fast the active diffuses through the excipient). The rate of diffusion through the excipient probably does not matter much, however, once the implant reaches steady state (*e.g.*, a constant or near constant elution rate). Thus, it may be desirable to have a higher ratio of soft segments relative to hard segments (*e.g.,* at least about 2:1, at least about 3:1, or at least about 4:1). The relative content of the soft and hard segments may also be considered directly proportional on the molecular weights of both the soft and hard segments. In other words, for a given ratio, a higher molecular weight polymer for the soft segment results in a higher relative content of soft segments to hard segments.

The molecular weights of each of the soft and hard segments may be selected depending on the specific soft and hard segments selected. In particular, the size (*e.g.,* molecular weight) of the soft segment may impact the elution rate. For example, the soft block (*e.g.,* polyether) molecular weights may range from about 1000-12,000 daltons (daltons may be used interchangeably with g/mol for molecular weight). For the case of PTMO as the soft segment, the molecular weights may range from about 500-3000 daltons. In some cases, a higher molecular weight may be preferred (*e.g.,* about 2000-3000 daltons) in order to elevate elution, as compared to less than about 1000 daltons. For the case of PPO as the soft segment, the molecular weight may range from about 2000-12,0000 daltons, and again a higher molecular weight may be preferred to elevate elution rates. For the case of polyether-block amides, the molecular weight of the polyether block may vary from about 400 to about 3000 daltons and that of the polyamide block may vary from about 500 to about 5000 daltons. Without wishing to be bound to a particular theory, it is believed that by increasing the molecular weight of soft segments in the polymer, the content of hard segments is reduced providing for better dissolution and diffusion of the API through the excipient.

The Shore D hardness or Shore hardness of the polymer segments may also have an impact on the elution rates. In some cases, the Shore hardness may be inversely proportional to the elution rate (*e.g.,* a higher Shore hardness results in a lower elution rate). For example, in the case of polyether-block amides, a Shore hardness of 35 provides a lower elution rate as compared to a Shore hardness of 25. In one embodiment of the present invention, the excipient is substantially or completely non-porous, in that the polymer has a porosity or void percentage less than about 10%, about 5%, or about 1%, for example. In particular, the excipient is substantially non-porous in that there are no physical pores or macropores which would allow for egress of the API from the drug delivery composition. In another embodiment, the excipient is practically insoluble in water, which equates to one gram in > 10,000 mL of water. In another embodiment of the present invention, the drug delivery composition does not require erosion or degradation of the excipient *in vivo* in order to release the API in a therapeutically effective amount. Alternatively, the excipient is not substantially erodible and/or not substantially degradable *in vivo* for the intended life of the implantable composition (*e.g.,* the API is not released due to erosion or degradation of the material *in vivo*).

The rate-controlling excipient may comprise a substantially non-porous, elastomeric polymer comprising soft and hard segments selected based on the relative content of soft and hard segments of the polymer to obtain an elution rate within a target range of average daily elution rate for the active pharmaceutical ingredient. A therapeutically effective amount of the API is delivered to the subject at a pseudo-zero order rate within a target range between a maximum and minimum value of a desired average daily elution rate for the active pharmaceutical ingredient. Pseudo-zero order refers to a zero-order, near-zero order, substantially zero order, or controlled or sustained release of the API. The composition may initially release an amount of the API that produces the desired therapeutic effect, and gradually and continually release other amounts of the API to maintain the level of therapeutic effect over the intended duration of treatment (*e.g.,* about one year).

As previously noted, the excipient defines the shape of the reservoir, which may be of any suitable size and shape. In an exemplary embodiment, the excipient is substantially cylindrically shaped. An embodiment of a cylindrically shaped excipient is depicted, for example, in Figure 2. The reservoir may be of any suitable size depending on the active and location of delivery, *e.g.,* a ratio of about 1:1.5 to 1:15, for example about 1:5 or about 1:10 diameter to length.

The wall thickness of the excipient may also be selected to provide for the desired elution rate. The wall thickness may be inversely proportional to elution rate. Thus, a larger wall thickness may result in a lower elution rate. The excipient may form a wall having an average thickness of about 0.05 to about 0.5 mm, or about 0.1 mm to about 0.3 mm (*e.g.,* about 0.1 mm, about 0.2 mm, or about 0.3 mm).

The polymers may be processed using any suitable techniques, such as extrusion, injection molding, compression molding, spin-casting. In one embodiment, a method of making an implantable drug delivery composition includes: (a) selecting a substantially non-porous elastomeric polymer comprising soft and hard segments based on the relative content and molecular weights of the soft and hard segments of the polymer to provide an elution rate within a target range of average daily elution rate for rasagiline; (b) forming a hollow tube from the elastomeric polymer (see *e.g.,* Figure 2); (c) selecting and formulating the rasagiline hemitartrate and at least one sorption enhancer in order to produce an elution rate at a therapeutically effective amount of the rasagiline at pseudo-zero order for a period of time of at least

one month, wherein the amount of sorption enhancer is directly proportional to the average daily elution rate; (d) loading at least one discrete solid dosage form comprising the rasagiline hemitartrate and the at least one sorption enhancer into the tube; and (e) sealing both ends of the tube to form a sealed cylindrical reservoir-based drug delivery composition. The tube may be sealed using any suitable means or techniques known in the art. For example, the ends may be plugged, filled with additional polymers, heat sealed, or the like. The tubes should be permanently sealed such that the discrete solid dosage forms may not be removed. Also, the ends should be suitably sealed such that there are no holes or openings that would allow egress of the active once implanted.

Sorption Enhancer(s) and the Discrete Dosage Form

According to an aspect of the present invention, the at least one discrete solid dosage form, within the reservoir, may also comprise at least one sorption enhancer in an amount effective to modulate the average daily elution rate of the active pharmaceutical ingredient to provide for release of the active pharmaceutical ingredient at pseudo-zero order within the target range at the therapeutically effective amount for a period of time of at least one month. As used herein, the terms "modulate" or "modulation" may be used to describe a change in the activity of the drug delivery composition. This may equate to a change in elution rate (*e.g.,* an increase or a decrease in a given elution rate or range).

Sorption enhancers may include compounds which improve the release of the API from the drug delivery composition. Without wishing to be bound to a particular theory, the sorption enhancers may improve release of the API from the drug delivery composition by drawing water or other fluids into the reservoir from the subject, disintegrating or breaking apart the discrete solid dosage form(s), and/or allowing the API to come into contact or remain in contact the inner walls of the excipient. Such a mechanism may be depicted, for example, in Figure 1.

The amount of the sorption enhancer is not particularly limited, but, when present, is preferably on the order of about 1-25 wt% of the solid dosage form, more preferably about 5-20 wt% of the solid dosage form, and more preferably about 10 wt%. The amount of sorption enhancer may be directly proportional to the elution rate. In other words, a higher weight percent of sorption enhancer in the composition may result in a higher average elution rate than a smaller weight percentage. Thus, it may be preferable to include a higher weight percent of sorption enhancer to give a higher elution rate (*e.g.*, about 8-25 wt% or about 10-20 wt%).

Any suitable sorption enhancer(s) may be selected by one of ordinary skill in the art. Particularly suitable sorption enhancer(s) may include, for example, negatively-charged polymers, such as croscarmellose sodium, sodium carboxymethyl starch, sodium starch glycolate, sodium acrylic acid derivatives (e.g., sodium polyacrylate), cross-linked polyacrylic acid (e.g., CARBOPOL®), chondroitin sulfate, poly-glutamic acid, poly-aspartic acid, sodium carboxymethyl cellulose, neutral polymers, such as polyethylene glycol, polyethylene oxide, polyvinylpyrrolidone, and combinations thereof. In an exemplary embodiment, the sorption enhancer is croscarmellose sodium. The amount of the sorption enhancer is not particularly limited, but, when present, is preferably on the order of about 1-25 wt% of the solid dosage form, about 2-20 wt% of the solid dosage form, about 2-12 wt% of the solid dosage form, about 5-10 wt% of the solid dosage form (*e.g.*, about 5 wt% or about 10 wt% of the solid dosage form). The selection of the specific sorption enhancer may have an impact on the elution rate.

In one embodiment of the present invention, the at least one discrete solid dosage form comprises: 75-97 wt% rasagiline hemitartrate based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. For example, 85-95 wt% (*e.g.,* about 88 wt%) rasagiline hemitartrate based on the total weight of the at least one discrete solid dosage form; 5-20 wt% (*e.g.,* about 10 wt%) of at least one sorption enhancer (*e.g.,* croscarmellose sodium) based on the total weight of the at least one discrete solid dosage form; and 0-5 wt% (*e.g.*, 2%) lubricant (*e.g.*, stearic acid) based on the total weight of the at least one discrete solid dosage form. Preferably, each component of the drug delivery composition is provided in an amount effective for the treatment of the disease or condition being treated.

As previously discussed, the therapeutically effective amount of the API may be delivered to the subject at a target range of average daily elution rate for the API. The target elution rate (mg/day) is based on the oral dose rate multiplied by the fractional oral bioavailability. The elution rate may be an average rate, *e.g.,* based on the mean average for a given period of time, such as a day (i.e., average daily elution rate). The average daily elution rate of the active pharmaceutical ingredient may vary in direct proportion to the amount of sorption enhancer in the drug delivery composition (*e.g.*, more sorption enhancer may provide for a higher average daily elution rate).

As previously discussed, the minimum value(s) for the average daily elution rate may be correlated to the trough value for an oral dosage version of the API (*e.g.*, based on the blood/plasma concentrations for oral formulations). Similarly, the maximum value(s) may be correlated to the peak value for an oral dosage version of the API (*e.g.*, the maximum blood/plasma concentration when an oral dosage is first administered or a pharmaceutically toxic amount). In other words, the target range is between maximum and minimum elution rates, respectively, which may be determined based on blood/plasma concentrations for equivalent oral dosage forms containing the same active. The number and

shape of the discrete dosage form(s) may be optimized to provide for the desired elution rates. For example, the discrete solid dosage forms may be of suitable shape to not fill the entire cavity of the reservoir. In one embodiment, the at least one discrete dosage form is cylindrical in shape. In another embodiment, the at least one discrete dosage form is substantially spherical in shape in that the solid dosage forms are spherical or nearly spherical. For example, the shape of the dosage form may not deviate from a perfect sphere by more than about 10%, In another embodiment, the at least one discrete dosage form is substantially cylindrical.

Without wishing to be bound by any theory, it is believed that the surface area of the at least one discrete dosage forms contributes to the elution rate. In one embodiment, the total surface area of the at least one discrete dosage forms is directly proportional to elution rate. Thus, the number of discrete dosage forms may be selected to provide a given elution rate, wherein an increased number of dosage forms provides an increased total surface area. The discrete solid dosage forms may comprise more than one pellet (*e.g.,* 2-9 pellets). In other words, a higher number of dosage forms may result in a higher average elution rate than a smaller number of dosage forms. Thus, it may be preferable to include more discrete solid dosage forms to give a higher elution rate *(e.g.,* 7-9 pellets). See *e.g.,* Figure 6 showing elution rates (μg/day) for rasagiline with reservoirs containing 7 or 9 pellets, where a composition containing 9 pellets produced a higher elution profile than a composition containing 7. In a further embodiment, the overall surface area of the pellets used in the implantable drug delivery composition can be increased, for example by changing the shape of the pellets, increasing their surface convolution, etc.

Drug Delivery Compositions, Subcutaneous Delivery Systems, and Kits

As previously noted, the drug delivery composition is long lasting, and the rasagiline may be delivered to the subject at a pseudo-zero order rate for an extended period of time (*e.g.,* at least about one month (about one month or greater), at least about three months (about three months or greater), at least about six months (about six months or greater), at least about one year (about one year or greater), at least about two years (about two years or greater), at least about 30 months (about 30 months or greater), or any period of time within those ranges).

According to one embodiment of the present invention, a subcutaneous delivery system for releasing rasagiline at a pseudo-zero order comprises an elastomeric reservoir implant comprising a rate-controlling excipient defining a reservoir. The rate-controlling excipient comprises a substantially non-porous elastomeric polymer having a relative content of hard segments and soft segments to provide an elution rate within a target range of average daily elution rate for the rasagiline. The reservoir containing at least one discrete solid dosage form comprising rasagiline hemitartrate and an effective amount of at least one sorption enhancer to modulate the elution rate of the rasagiline for release of a therapeutically effective amount of the rasagiline within the target range at pseudo-zero order for a period of time of at least one month. The amount of sorption enhancer may be directly proportional to the average daily elution rate.

The drug delivery composition may be implanted into the subject in any suitable area of the subject using any suitable means and techniques known to one of ordinary skill in the art. For example, the composition may be implanted subcutaneously, *e.g.,* at the back of the upper arm or in the upper back (*e.g.*, scapular region), by directly depositing in or underneath the skin, a subcutaneous fat layer, or intramuscularly.

According to another embodiment of the present invention, a kit for subcutaneously placing a drug delivery composition includes a reservoir-based drug delivery composition comprising a rate-controlling excipient defining a reservoir containing at least one discrete solid dosage form and an implanter for inserting the reservoir-based drug delivery composition beneath the skin, and optionally instructions for implantation and explantation of the drug delivery composition. The rate-controlling excipient comprises a substantially non-porous, elastomeric polymer comprising soft and hard segments and the relative content of soft and hard segments of the polymer are selected to obtain an elution rate within a target range of average daily elution rate for the rasagiline. The at least one discrete solid dosage form preferably comprises rasagiline hemitartrate and at least one sorption enhancer in an amount effective to modulate the elution rate of the rasagiline to provide for release of the rasagiline at pseudo-zero order within the target range at the therapeutically effective amount for a period of time of at least one month, and the amount of sorption enhancer may be directly proportional to the average daily elution rate.

The drug delivery composition may be delivered subcutaneously using any suitable equipment or techniques, *e.g.,* an implanter known to one ordinary skill in the art. The kits may comprise the drug delivery composition pre-loaded into the implanter or the drug delivery composition may be loaded by the doctor or other user. The implanter may be an implantation device, such as a syringe, cannula, trocar or catheter, that may be inserted into an incision made at the delivery site of the subject. Suitable implantation devices and implantation methods include the trocar and methods disclosed in US 7,214,206 and US 7,510,549, the disclosures of which are herein incorporated by reference in their entirety, for all purposes. Other suitable methods for implanting or otherwise positioning the compositions into the body, *e.g.,* by a doctor, are well known in the art. Removal and/or replacement may also be accomplished using suitable tools and methods known in the art. Kits may also comprise other equipment well known in the art, such as scalpels, clamps, suturing tools, hydration fluid, and the like.

Embodiments of Kits and Methods of Use Thereof

As used herein, the terms "proximal" and "distal" refer respectively to the directions closer to and further from the surgeon implanting the drug-eluting implant. For purposes of clarity, the distal portion of the insertion instrument is inserted into a subject and the proximal portion of the instrument remains outside the subject. For frame of reference in the figures, arrows marked "P" refer generally to the proximal direction and arrows marked "D" refer generally to the distal direction relative to the orientation of the items depicted in the figures.

Referring to Figure 8, a kit 10 for subcutaneously placing a drug-eluting implant in a subject is shown in accordance with one exemplary embodiment of the invention. Kit 10 includes a drug-eluting implant 100 and an insertion instrument 200 for subcutaneously placing the drug-eluting implant in a subject. Insertion instrument 200 is packaged with implant 100 pre-loaded into the insertion instrument 200. Although insertion instrument 200 is shown with a single drug-eluting implant 100, the instrument may be pre-loaded with two or more drug-eluting implants to be implanted into a subject. In addition, one or more drug-eluting implants 100 may be provided in kit 10 that are packaged separately from insertion instrument 200.

Referring to Figures 9-17, insertion instrument 200 includes a cannula 210 having a hollow shaft 230 where the cannula 210 connects to a front hub portion 223 of a handle portion 224 of the insertion instrument 200. The cannula and hence the hollow shaft 230 has a longitudinal axis 240 and forms an interior bore or lumen 232 that extends through the hollow shaft. The cannula 210 has a sharp distal end 234 that may be covered by a protective sheath 231, shown in Figure 9, when insertion instrument 200 is not in use. Insertion instrument 200 also includes a stop rod 250 capable of extending through (i) a rear hub portion 220 of the handle portion 224, (ii) the handle portion 224, (iii) the front hub portion 223 of the handle portion 224, and (iv) into hollow shaft 230. Cannula 210 is slidably displaceable over stop rod 250, as will be described in more detail.

In accordance with embodiments of the invention, the handle portion 224 may be formed with a number of different constructs. For example, handle portion 224 may be constructed from two injection molded portions 220a and 220b. Portions 220a and 220b may connect to one another with, for example, a plurality of pins (not shown) that mate with a corresponding plurality of sockets 228 (shown in Figure 17). When portions 220a and 220b are connected with one another, they collectively form the rear hub portion 220 and the front hub portion 223 of the handle portion 224, and the handle portion 224. As will be readily apparent to those skilled in the art, other constructions are possible for handle portion 224. Front hub portion 223 is adapted to receive the cannula 210 and stop rod 250 therein. Handle portion 224 is offset to one side of longitudinal axis 240 of hollow shaft 230, forming a lateral extension that can be gripped by the user. A pair of flanges 221 project outwardly from handle portion 224 for engagement with a user's fingers.

Distal end 234 of hollow passage 230 provides a passageway into lumen 232. Lumen 232 is adapted to receive and store drug-eluting implant 100 inside hollow shaft 230. Drug-eluting implant 100 can be loaded into lumen 232 by inserting the implant through open distal end 234 and into hollow shaft 230. In this arrangement, drug-eluting implant 100 can be pre-loaded into insertion instrument 200 by the manufacturer after the instrument 200 is assembled. Alternatively, drug-eluting implant 100 can be loaded into insertion instrument 200 by the user.

Referring to Figure 17, insertion instrument 200 is shown in a ready-to-use condition, with drug-eluting implant 100 pre-loaded into hollow shaft 230 of the cannula 210. Stop rod 250 includes a proximal end 252 and a distal end 254. Proximal end 252 of stop rod 250 includes a knob or handle portion 256. Distal end 254 of stop rod 250 includes an abutment face 259. Abutment face 259 is disposed within hollow shaft 230 in close proximity to drug-eluting implant 100.

Cannula 210 is slidably displaceable over stop rod 250, as noted above. Insertion instrument 200 has two settings, one which allows axial displacement of the cannula 210 over stop rod 250, and one that prevents axial displacement. The settings are controlled by the relative orientation of stop rod 250 with respect to cannula 210. Stop rod 250 is axially rotatable relative to longitudinal axis 240 of hollow shaft 230 between an unlocked orientation and a locked orientation. In the unlocked orientation, cannula 210, front hub 223 and rear hub 220 are permitted to slide over stop rod 250. In the locked orientation, cannula 210, front hub 223 and rear hub 220 are prevented from sliding over stop rod 250.

Stop rod 250 includes a first locking feature defined by two longitudinal grooves 236 as best seen in Figure 9A. Grooves 236 extend along a portion of the length of stop rod 250. Handle portion 224 includes a second locking feature defined by a pair of projections 216 located on rear hub 220 as best seen in Figure 17. Each projection 216 extends radially inwardly toward horizontal axis 240 of the hollow shaft 230. When stop rod 250 is rotated into the locked orientation, grooves 236 are not in radial alignment with projections 216. As such, projections 216 engage stop rod 250, preventing cannula 210 from sliding over the stop rod toward proximal end 252 of the stop rod. When stop rod 250 is rotated to the unlocked orientation, grooves 236 are positioned in radial alignment with projections 216. Each groove 236 is sized to receive one of the projections 216. Therefore, in the unlocked position, each projection 216 is received within a groove 236 thereby permitting the cannula 210 to slide over stop rod 250 toward proximal end 252 of the stop rod 250. Stop rod 250 may include spaced markings thereon to indicate the distance that the cannula 210 has been moved proximally with respect to the proximal end 252 of the stop rod 250.

Insertion instrument 200 is packaged in the kit 10 with the drug-eluting implant 100 pre-loaded into the cannula

210. In alternative embodiments, the kit may be provided with an insertion instrument 200 and a drug-eluting implant 100, with the implant packaged separately from the instrument (i.e. the instrument is contained in one package in the kit, and the implant is contained in a separate package in the kit outside of the package containing the instrument). This packaging option allows a user to remove the drug-eluting implant from its packaging, inspect the implant, and load the implant into the instrument immediately before inserting the implant into the patient. This option also provides the user with the flexibility to substitute the implant provided in the kit with another implant that may be more suitable. Separate packaging may be used with kits that contain multiple implants having different properties. In such kits, the different implants may be individually packaged, and the user may select and open the appropriate implant, and load that implant into the instrument.

Kits in accordance with the invention may contain one or more implants that differ from one another in terms of the drug composition they contain, or other characteristic. For example, kit 10 is provided with a single drug-eluting implant 100. Implant 100 consists of a polymeric rate-controlling excipient, the excipient defining a reservoir containing at least one discrete solid dosage form. Other kit embodiments may be provided with two or more implants consisting of polymeric rate-controlling excipients. Although the figures schematically show a single implant 100 pre-loaded in insertion instrument 200, other embodiments in accordance with the invention may feature insertion instruments pre-loaded with two or more implants 100. Kits in accordance with embodiments of the invention may be provided with an insertion instrument pre-loaded with one or more implants, and one or more separately packaged implants that are not pre-loaded in the insertion instrument. Any number, type or combination of implants and instruments, whether packaged together or separately, may be provided in kits in accordance with embodiments of the invention. Thus, multiple implants having different therapeutic effects may be implanted in a single delivery procedure.

It is desirable in some instances to prepare a subcutaneous cavity beneath the cutis, prior to inserting insertion instrument 200 into the subject. The subcutaneous cavity provides a pocket that is large enough to receive the full length of the hollow shaft of the cannula, making it easier to deposit the implant in the proper location. For this reason, kits in accordance with embodiments of the invention may optionally include a separate instrument for preparing a subcutaneous cavity in a subject. Referring to Figure 18, an alternate kit 10’ in accordance with embodiments of the invention is shown. Kit 10’ includes the same insertion instrument 200 pre-loaded with a drug-eluting implant 100 as shown in prior figures. Kit 10’ also includes a second instrument, referred to as a tunneling instrument 300, for preparing a subcutaneous cavity in a subject. In addition, kit 10’ includes another drug-eluting implant 100’ that is packaged separately from the instruments.

Referring to Figures 19-27, tunneling instrument 300 has an elongated profile characterized by a horizontal axis H that is parallel to an insertion direction I, and a vertical axis V that is normal to the horizontal axis. Tunneling instrument 300 includes a blade 310 and a handle 350 attached to the blade. Blade 310 has a proximal end 312 and a distal end 314. Handle 350 also has a proximal end 352 and a distal end 354. In the present embodiment, distal end 354 of handle 350 is attached to proximal end 312 of blade 310 by a pair of screws 311. As will be readily apparent to those skilled in the art, blade 310 may be attached to handle 350 by any other means known in the art. When blade 310 is viewed from a side, as shown in Figure 19, the vertical height or dimension of the blade 310 with respect to vertical axis V gradually increases from distal end 314 toward the proximal end 312. Blade 310 includes a superior surface 316 and an inferior surface 318 opposite the superior surface. Inferior surface 318 extends between the proximal and distal ends 312, 314 of blade 310 and includes a substantially flat portion 322 that extends parallel to horizontal axis H. Superior surface 316 of blade 310 forms an inclined surface 324. Inclined surface 324 extends at an acute angle $\theta$ (as best seen in Figure 20) with respect to flat portion 322. Referring to Figure 23, blade 310 has a tapered profile with a maximum width at proximal end 312. The width of blade 310 tapers to a minimum width at the distal end 314. Each side of blade 310 follows a gradual curve. Blade 310 may be covered by a protective sheath 315, as shown in Figure 22, when tunneling instrument 300 is not in use.

Handle 350 includes a base portion 356 and an elongated gripping portion 358 extending from the base portion. Base portion 356 has a superior surface 362 and an inferior surface 364 opposite the superior surface. Inferior surface 364 extends substantially coplanar with flat portion 322 of blade 310 to form a substantially continuous surface between the blade 310 and base portion 356. Gripping portion 358 extends upwardly from base portion 356 with respect to vertical axis V, and features a superior surface 366 and an inferior surface 368. An overmolded grip 372 extends over superior surface 366 of gripping portion 358 and superior surface 362 of base portion 362. Overmolded grip 372 may be formed of rubber or other material that provides a soft cushioned area to grip the instrument.

A method for subcutaneously placing a drug-eluting implant in a subject in accordance with embodiments of the invention will now be described with reference to the instruments in kit 10’. In this example, the method is used to subcutaneously place the implant in the arm of a human subject. The method begins by positioning the patient so that the surgeon has access to the location into which the implant is to be placed. For example, the patient may be positioned lying down on his or her back, with one arm flexed and turned to give the surgeon access to the inner aspect of the upper arm. The insertion site is then located on the upper arm. One possible insertion site is located approximately halfway between the patient’s shoulder and elbow, and in the crease between the bicep and triceps. Once the insertion site is selected, the area around the site is swabbed and a local anesthetic is administered. Using a sterile scalpel, the

surgeon makes an incision at the insertion site in a direction transverse to the long axis of the upper arm. The length of the incision should be as short as possible, but long enough to allow insertion of blade 310 of tunneling instrument 300 into the incision and under the skin. In alternate embodiments, the drug-eluting implant may be placed without the aid of a tunneling instrument. In such cases, the length of the incision should be as short as possible, but long enough to allow insertion of the cannula 210 of the insertion instrument 200 into the incision and under the skin.

For cases when a tunneling instrument 300 is used, the tunneling instrument 300 is removed from its packaging (if not already done) and placed in proximity to the incision, with flat portion 322 of blade 310 resting on or positioned just above the skin, and distal end 314 of the blade aligned with the incision. Inferior surface 364 of base portion 356 of handle 350 should also be resting on or positioned just above the skin, so that flat portion 322 of blade 310 is substantially parallel to the long axis of the patient's arm. Distal end 314 of blade 310 is then inserted through the incision and advanced into the patient's arm in a direction substantially parallel to the long axis of the arm, with the blade advancing immediately beneath the cutis and into the subcutaneous tissue. As blade 310 is advanced into the arm, the portion of the blade that enters the arm becomes gradually wider and wider in the horizontal and vertical directions due to the geometry of the blade 310 discussed above to expand the cavity created by the blade, forming a pocket or tunnel by blunt dissection. During insertion, the surgeon preferably maintains the insertion path just beneath the cutis and visibly raises the skin with blade 310 until a subcutaneous tunnel of sufficient length and width is created. Blade 310 is then removed from the patient's arm. For single-use kits, tunneling instrument 300 may be discarded.

Insertion instrument 200 is then removed from its packaging (if not already done). As noted above, insertion instrument 200 is packaged in kit 10' with drug-eluting implant 100 pre-loaded into cannula 210. Insertion instrument 200 is preferably packaged with stop rod 250 withdrawn from handle portion 224 and in the locked position as shown in Figure 8. Prior to use, the surgeon may wish to check that insertion instrument 200 is set with stop rod 250 rotated to the locked position, so as to prevent cannula 210 from being inadvertently advanced over the stop rod 250. The surgeon can determine if stop rod 250 is locked in a number of ways. For example, the surgeon can try sliding the cannula 210 over stop rod 250 to see if the stop rod is locked or unlocked. In addition, or as an alternative, the surgeon can check visible markings on insertion instrument 200 to determine whether stop rod 250 is locked or unlocked. In the illustrated example, rear hub portion 220 has a first indicia 222 in the form of a small horizontal line (as best seen in Figures 13 and 14). Stop rod 250 has a second indicia 251 and a third indicia 253 in the form of two horizontal lines that are radially offset from one another on the perimeter of the stop rod (as best seen in Figure 13). Stop rod 250 is rotatable relative to hub 220 to a first orientation that aligns the second indicia 251 with the first indicia 222. This first orientation corresponds to the locked position. Stop rod 250 is also rotatable relative to the hub 220 to a second orientation that aligns the third indicia 253 with the first indicia 222. This second orientation corresponds to the unlocked position. In preferred embodiments, the instrument includes a mechanism that provides tactile feedback to the surgeon when the stop rod 250 is rotated to the locked and unlocked positions. For example, the instrument may include an internal spring latch that engages a detent inside the hub to make an audible click after the stop rod is rotated to the locked position and/or unlocked position. The second and third indicia may also be color coded (e.g. green and red lines) to suggest which orientation is the unlocked position and which orientation is the locked position.

Once the locked position is confirmed, distal end 234 of cannula 210 is inserted into the incision and advanced into the subcutaneous tissue. Cannula 210 is advanced into the tunnel until a distal end 229 of hub 220 reaches the incision. At this stage, the hollow shaft 230 and hence, the implant 100, is positioned in the tunnel. Stop rod 250 is then rotated to the unlocked position in preparation for withdrawing cannula 210 from the incision. The unlocked position can be confirmed by an audible click, or by visual reference using the first indicia 222 and third indicia 253. The surgeon applies a gentle downward pressure on top of stop rod 250, preferably at or near proximal end 252, so as to fix the position of the stop rod relative to the patient's arm. Once stop rod 250 is fixed, the surgeon holds the stop rod 250 in the fixed position with one hand, and grasps the handle portion 224 of the insertion instrument 200 with the other hand. The surgeon then applies a pulling force on handle portion 224 in a direction away from the incision to withdraw cannula 210 out of the incision. This may be performed in a single rapid motion to withdraw cannula 210 from the tunnel while leaving implant 100 in place in the tunnel. Depending on the length of implant 100 relative to the length of cannula 210 and other factors, the implant may be completely released from the hollow shaft 230 when the cannula 210 is partially removed from the incision (i.e. when a portion of the cannula 210 is withdrawn from the tunnel, while the remaining portion of the cannula 210 still remains in the tunnel). In other scenarios, implant 100 may be completely released from hollow shaft 230 only after the entire cannula 210 is completely removed from the incision (i.e. no portion of the cannula 210 remains in the tunnel).

Depending on factors such as friction, implant 100 may travel a small distance with cannula 210 as the cannula is withdrawn from the tunnel. In the event that implant 100 travels with cannula 210, the implant may travel far enough to contact abutment face 259 of stop rod 250. Abutment face 259 remains fixed inside the tunnel as cannula 210 is withdrawn, preventing the implant from being pulled out of the tunnel as the cannula 210 is withdrawn and removed from the incision.

In another embodiment, the implant 100 may be delivered as follows. Once the locked position is confirmed,

distal end 234 of cannula 210 is inserted into the incision and advanced into the subcutaneous tissue. Cannula 210 is advanced into the tunnel until the distal end 234 of the cannula 210 is at the desired location of implant delivery in the tunnel. At this stage, the stop rod 250 is then rotated to the unlocked position in preparation for advancing the implant 100 toward the distal end 234 of the cannula 210. Similar to the previous embodiment, the unlocked position can be confirmed by an audible click, or by visual reference using the first ind icia 222 and third indicia 253. The surgeon next pushes the stop rod 250 distally thereby advancing the implant 100 in the hollow shaft 230 toward the distal end 234 of the cannula 210. Once the implant is at the distal end 234, the surgeon then applies a gentle downward pressure on top of stop rod 250, preferably at or near proximal end 252, so as to fix the position of the stop rod relative to the patient's arm. Once stop rod 250 is fixed, the surgeon holds the stop rod 250 in the fixed position with one hand, and grasps the handle portion 224 of the insertion instrument 200 with the other hand. The surgeon then applies a pulling force on handle portion 224 in a direction away from the incision to withdraw cannula 210 out of the incision. Moving the handle portion 224 and hence, the cannula 210 in this manner while holding the stop rod 250 and hence, the implant 100, stationary, causes the implant 100 to be delivered out of the hollow shaft 230 and into the subject.

Once cannula 210 is withdrawn from the tunnel, the surgeon can check the position of implant 100 inside the tunnel. The surgeon can confirm proper placement of implant 100 by palpation and inspection of the incision. After correct placement is confirmed, the surgeon or other medical professional should cover the insertion site with sterile gauze, apply pressure to the insertion site, and follow any other post-operative procedures that are required.

To remove implant 100, an incision is made transverse to the long axis of the upper arm adjacent to one end of the implant. The incision should be of a size adequate to allow the tips of a hemostat to enter the tunnel. The tips of the hemostat are inserted into the incision and positioned on opposite sides of implant 100 in a position to grasp the implant. Implant 100 is then grasped and carefully pulled out of the pocket. After implant 100 is removed, the surgeon or other medical professional should cover the insertion site with sterile gauze, apply pressure to the insertion site, and follow any other post-operative procedures that are required.

Many elements shown in the illustrated embodiments are ornamental elements. The appearance of each ornamental element is not dictated by any function that the feature may perform. Rather, the appearance of each ornamental feature is selected based on aesthetic considerations. These ornamental elements may have a wide variety of shapes, colors, dimensions and surface textures that are selected individually, or in combination, to achieve a desired product appearance. For example, the shape, contours and relative dimensions of flanges 221 on insertion instrument 200 need not be as shown in Figures 8-16, which show the flanges as crescent-shaped elements. Flanges 221 may be larger or smaller, and/or have other shapes such as triangular or rectangular shapes, without changing any functional aspects of insertion instrument 200. Other ornamental aspects of insertion instrument 200 include, but are not limited to, the circular perimeter of handle portion 224 (which can be any shape), the common border between the circular perimeter of the handle portion and the perimeter of each flange, the rounded transitions between the handle portion and front hub 223, the off-centered axial position of the handle portion with respect to the front hub 223, and the differences in length and diameter among the various parts of the hub and stop rod. The tunneling tool 300 also has many ornamental features, including but not limited to the compound curvatures on superior surface 366 of gripping portion 358, the compound curvatures on inferior surface 368 of the gripping portion, the hourglass shaped profile of the gripping portion (Figure 23), the curved sides and rounded corners of overmolded grip 372 (Figures 19 and 20), the U-shape of base section 356 (Figures 21-23), and the contrasting surface texture between overmolded grip 372 and gripping portion 358. These ornamental aspects of the embodiments, which are only some of the ornamental aspects shown on the embodiments, do not influence the utilitarian aspects of the instruments or the functional purposes of any parts, and therefore may be replaced by an infinite number of other ornamental designs.

EXAMPLES

Embodiments of the present invention may be further understood by reference to the Examples provided below.

**Example 1: Rasagiline + Sorption Enhancer**

The follow general procedure was followed for the manufacture of an implant containing rasagiline. Tubing was received in continuous length rolls and was cut to an appropriate starting length using a single-edged razor blade (or suitably sized scalpel). One end of each tubing section was thermally sealed, imparting a semi-spherical closure on the tip of the tubing section.

A discrete solid dosage form was prepared as follows. Rasagiline salt and a sorption enhancer, croscarmellose sodium, were premixed in a Turbula blender. Stearic acid was added as a lubricant and the mixture was again mixed in a Turbula blender. The standard drug blend was 88% rasagiline salt, 10% croscarmellose sodium, and 2% stearic acid.

The drug blend was compacted using a single punch tablet press. Drug pellets were manually placed inside each sealed section of tubing. The open section of each pellet-containing tubing section was then sealed into a semi-spherical seal. Sterilization was accomplished by gamma irradiation of the implants.

**Example 2: Rasagiline Hemitartrate Implants**

Drug implants were manufactured as described in Example 1 using the following tubing materials: Tecoflex® EG-80A and Tecoflex® EG85A, polyurethanes with a polyether soft segment of MW 2,000; Tecoflex® EG-93A, a polyurethane with a polyether soft segment of MW 1,000; and two different PEBAX® polymers, 2533 and 3533, a polyamide with a polyether soft segment. The implant dimensions were a total length of the implant of about 35 mm, an outer diameter (O.D.) of 4.0 mm, an inner diameter (I.D.) of 3.6 mm and a wall thickness of 0.2 mm. A total of about 272 mg of the rasagiline hemitartrate blend (i.e., including 10% croscarmellose and 2 % stearic acid and 240 mg rasagiline) were loaded into each implant by placing 5 drug pellets into each implant. The implants were sterilized by gamma irradiation and placed in an elution batch consisting of 50 mL 0.9% saline at 37°C. Weekly exchanges of the elution media were analyzed by HPLC for 21 weeks. The graph of elution rate vs. "pull day" is shown in Figure 4. The pull day is the day on which the elution media was sampled and analyzed. Figure 4 depicts the elution rates of rasagiline from an aliphatic, polyether-based urethane implant over 21 weeks.

**Example 3: Rasagiline Mesylate Implants**

A drug implant was manufactured as described in Example 1 using Tecoflex® EG-80A, a polyurethane with a polyether soft segment, as the tubing material and rasagiline mesylate as the API salt. The implant dimensions were a total length of the implant of about 45 mm, an outer diameter (O.D.) of 4.0 mm, an inner diameter (I.D.) of 3.6 mm and a wall thickness of 0.2 mm. A total of about 440 mg of the rasagiline mesylate blend (i.e., including 10% croscarmellose and 2 % stearic acid and 385 mg rasagiline) were loaded into the implant. The implant was sterilized by gamma irradiation and placed in an elution batch consisting of 50 mL 0.9% saline at 37°C. Weekly exchanges of the elution media were analyzed by HPLC over 70 days. The graph of elution rate vs. "pull day" is shown in Figure 5. The pull day is the day on which the elution media was sampled and analyzed. Figure 5 depicts the elution rate of rasagiline mesylate from the implant over about 10 weeks. The elution started high and dropped consistently over the 9 weeks of the experiment, and the implants started to swell at week 4 and burst between week 9 and week 10. The implants never achieved controlled release of rasagiline in contrast to the implants shown in Example 2.

**Example 4: Rasagiline Hemitartrate Implant**

The drug implants were manufactured as described in Example 1 using Tecoflex® EG-80A, a polyurethane with a polyether soft segment of MW 2,000. The implants had an outer diameter (O.D.) of 4.0 mm, an inner diameter (I.D.) of 3.6 mm and a wall thickness of 0.2 mm. About 340 mg and about 440 mg of the rasagiline hemitartrate blend (i.e., each with 10% croscarmellose and 2 % stearic acid and about 300 mg and 385 mg rasagiline, respectively) were loaded into each implant by placing 7 pellets and 9 pellets into each implant, respectively. The implants were sterilized by gamma irradiation and placed in an elution batch consisting of 50 mL 0.9% saline at 37 °C. Weekly exchanges of the elution media were analyzed by HPLC for over 31 weeks. The graph of elution rate vs. "pull day" is shown in Figure 6. The pull day is the day on which the elution media was sampled and analyzed. The graph in Figure 6 demonstrates pseudo-zero order release from the implants and that the release rate can be controlled by the amount of API blend loaded into the implant.

**Example 5: *In Vivo* Implant Testing**

Three beagle dogs were dosed orally with Azilect® (rasagiline mesylate) for 10 days to establish peak and trough plasma concentration levels of rasagiline in beagles. In Figure 7, the initial dose is shown by the line from Day 0 to about Day 10. Due to the very short half-life of rasagiline of about 3 hours, rasagiline levels are either undetectable or very low. The last dose is administered at about Day 10, followed by a 3 day wash-out period. Average peak plasma concentration levels were approximately 1 ng/mL. After the 3 day wash-out period, the same beagles, serving as their own controls, were then implanted with a rasagiline hemitartrate implant containing about 300 mg rasagiline, prepared as described in Example 2. As shown in Figure 7, the implantation occurred on about Day 13 with average plasma concentrations of about 2 - 4 ng/mL, reached within 1 day after implantation. The level of rasagiline was maintained throughout the experiment for the evaluation period of about 114 days (about 16 weeks).

## Claims

1. A drug delivery composition comprising:

   a drug elution rate-controlling excipient comprising an elastomeric polymer defining a reservoir, and
   the reservoir contains at least one discrete solid dosage form comprising rasagiline hemitartrate,
   wherein the drug delivery composition is in an implantable dosage form.

2. The drug delivery composition according to claim 1, wherein the elastomeric polymer is a thermoplastic elastomer comprising polyurethane-based polymers, polyether-based polymers, polysilicone-based polymers, polycarbonate-based polymers, or combinations thereof and/or wherein the elastomeric polymer comprises a polyether-based polyurethane.

3. The drug delivery composition according to claim 1, wherein the polyether-based polyurethane is an aliphatic polyether-based polyurethane comprising poly(tetramethylene oxide) and polymerized 4,4'-diisocyanato dicyclohexylmethane (H12MDI) and 1,4-butanediol.

4. The drug delivery composition according to claim 3, where the polyether-based polyurethane comprises a Shore hardness less than 87A.

5. The drug delivery composition according to claim 1, wherein the elastomeric polymer comprises a polyether amide and/or
   wherein the at least one discrete solid dosage form is cylindrical.

6. The drug delivery composition according to claim 1, wherein the reservoir contains 5-10 discrete solid dosage forms.

7. The drug delivery composition according to claim 6, wherein the discrete solid dosage forms comprise about 200 mg to about 500 mg of the rasagiline hemitartrate.

8. The drug delivery composition according to claim 1, wherein the drug elution rate-controlling excipient is cylindrically shaped, and/or
   wherein the at least one discrete solid dosage form comprises at least one sorption enhancer selected from the group consisting of croscarmellose sodium, sodium carboxymethyl starch, sodium starch glycolate, sodium acrylic acid derivatives, chondroitin sulfate, poly-glutamic acid, poly-aspartic acid, and combinations thereof.

9. A drug delivery composition for use as a medicament in a method of treating one or more symptoms of Parkinson's disease comprising:

   implanting a reservoir-based drug delivery composition into a subject to systemically deliver a therapeutically effective amount of rasagiline to the subject for a period of time of at least one month,
   wherein the drug delivery composition comprises at least one discrete solid dosage form comprising rasagiline hemitartrate surrounded by an excipient comprising at least one polymer.

10. The drug delivery composition for use according to claim 9,
    wherein the at least one discrete solid dosage form comprises:

    75-97 wt% rasagiline hemitartrate based on the total weight of the at least one discrete solid dosage form;
    1-25 wt% of at least one sorption enhancer based on the total weight of the at least one discrete solid dosage form; and
    0-5 wt% lubricant based on the total weight of the at least one discrete solid dosage form.

11. The drug delivery composition for use according to claim 9,
    wherein the therapeutically effective amount of the rasagiline is delivered at a pseudo-zero order rate and/or
    wherein the drug delivery composition does not require erosion or degradation of the excipient *in vivo* to release the rasagiline in the therapeutically effective amount.

12. The drug delivery composition for use according to claim 9,
    wherein the therapeutically effective amount of the rasagiline is delivered to the subject at a target range of about

100 to about 1000 micrograms/day and/or
wherein the method is used as monotherapy for treating the subject’s symptoms of Parkinson’s disease.

**13.** The drug delivery composition for use according to claim 9,
wherein the method is used as adjunctive therapy in addition to one or more other dopaminergic medications and/or wherein the at least one polymer is a thermoplastic elastomer comprising polyurethane-based polymers, polyether-based polymers, polysilicone-based polymers, polycarbonate-based polymers, or combinations thereof and/or wherein the at least one polymer comprises a polyether-based polyurethane.

**14.** A subcutaneous delivery system according to claim 1 comprising:

an elastomeric reservoir implant comprising at least one discrete solid dosage form surrounded by a polymeric rate-controlling excipient,
the at least one discrete solid dosage form comprising rasagiline hemitartrate,
wherein the subcutaneous delivery system provides for release of the rasagiline at an elution rate suitable to provide a therapeutically effective amount of the rasagiline to a subject at a zero order or pseudo-zero order rate for a period of time of at least one month.

**15.** A kit for subcutaneously placing a drug delivery composition comprising:

a reservoir-based drug delivery composition according to claim 1 comprising a polymeric rate-controlling excipient defining a reservoir containing at least one discrete solid dosage form comprising rasagiline hemitartrate; and
an implanter for inserting the reservoir-based drug delivery composition beneath the skin.

## Patentansprüche

**1.** Arzneimittelabgabezusammensetzung, die Folgendes umfasst:

einen die Arzneimittel-Elutionsrate regulierenden Hilfsstoff, der ein elastomeres Polymer umfasst, das ein Reservoir definiert, und
das Reservoir wenigstens eine diskrete feste Darreichungsform enthält, die Rasagilin-Hemitartrat umfasst,
wobei die Arzneimittelabgabezusammensetzung in einer implantierbaren Darreichungsform vorliegt.

**2.** Arzneimittelabgabezusammensetzung nach Anspruch 1, wobei das elastomere Polymer ein thermoplastisches Elastomer ist, das Polymere auf Polyurethanbasis, Polymere auf Polyetherbasis, Polymere auf Polysilikonbasis, Polymere auf Polycarbonatbasis oder Kombinationen davon umfasst, und/oder wobei das elastomere Polymer ein Polyurethan auf Polyetherbasis umfasst.

**3.** Arzneimittelabgabezusammensetzung nach Anspruch 1, wobei das Polyurethan auf Polyetherbasis ein aliphatisches Polyurethan auf Polyetherbasis ist, das Poly(tetramethylenoxid) und polymerisiertes 4,4’-Diisocyanatdicyclohexylmethan (H12MDI) und 1,4-Butandiol umfasst.

**4.** Arzneimittelabgabezusammensetzung nach Anspruch 3, wobei das Polyurethan auf Polyetherbasis eine Shore-Härte von weniger als 87 A hat.

**5.** Arzneimittelabgabezusammensetzung nach Anspruch 1,
wobei das elastomere Polymer ein Polyetheramid umfasst und/oder
wobei die wenigstens eine diskrete feste Darreichungsform zylindrisch ist.

**6.** Arzneimittelabgabezusammensetzung nach Anspruch 1, wobei das Reservoir 5-10 diskrete feste Darreichungsformen enthält.

**7.** Arzneimittelabgabezusammensetzung nach Anspruch 6, wobei die diskreten festen Darreichungsformen etwa 200 mg bis etwa 500 mg des Radagilin-Hemitartrats umfassen.

**8.** Arzneimittelabgabezusammensetzung nach Anspruch 1, wobei der Arzneimittel-Elutionsrate regulierende Hilfsstoff

zylindrisch geformt ist, und/oder
wobei die wenigstens eine diskrete feste Darreichungsform wenigstens einen Resorptionsenhancer umfasst, ausgewählt aus der Gruppe bestehend aus Croscarmellose-Natrium, Natriumcarboxymethyl-Stärke, Natriumstärke-Glycolat, Natriumacrylsäurederivaten, Chondroitinsulfat, Poly-Glutaminsäure, Poly-Asparaginsäure und Kombinationen davon.

**9.** Arzneimittelabgabezusammensetzung zur Verwendung als Medikament in einem Verfahren zur Behandlung von einem oder mehreren Symptomen der Parkinson-Krankheit, das Folgendes beinhaltet:

Implantieren einer Arzneimittelabgabezusammensetzung auf Reservoir-Basis in ein Subjekt zum systematischen Abgeben einer therapeutisch wirksamen Menge Rasagilin an das Subjekt für eine Zeitperiode von wenigstens einem Monat,
wobei die Arzneimittelabgabezusammensetzung wenigstens eine diskrete feste Darreichungsform umfasst, die Rasagilin-Hemitartrat umfasst, das von einem Hilfsstoff umgeben ist, der wenigstens ein Polymer umfasst.

**10.** Arzneimittelabgabezusammensetzung zur Verwendung nach Anspruch 9, wobei die wenigstens eine diskrete feste Darreichungsform Folgendes umfasst:

75-97 Gew.-% Rasagilin-Hemitartrat auf der Basis des Gesamtgewichts der wenigstens einen diskreten festen Darreichungsform;
1-25 Gew.-% von wenigstens einem Resorptionsenhancer auf der Basis des Gesamtgewichts der wenigstens einen diskreten festen Darreichungsform; und
0-5 Gew.-% Gleitmittel auf der Basis des Gesamtgewichts der wenigstens einen diskreten festen Darreichungsform.

**11.** Arzneimittelabgabezusammensetzung zur Verwendung nach Anspruch 9,
wobei die therapeutisch wirksamen Menge des Rasagilins mit einer Ordnungsrate Pseudo-Null abgegeben wird, und/oder
wobei die Arzneimittelabgabezusammensetzung keine(n) Erosion oder Abbau des Hilfsstoff in vivo erfordert, um das Rasagilin in der therapeutisch wirksamen Menge freizusetzen.

**12.** Arzneimittelabgabezusammensetzung zur Verwendung nach Anspruch 9,
wobei die therapeutisch wirksamen Menge des Rasagilins dem Subjekt in einem Zielbereich von etwa 100 bis etwa 1000 Mikrogamm/Tag abgegeben wird, und/oder
wobei das Verfahren als Monotherapie zur Behandlung von Symptomen der Parkinson-Krankheit bei dem Subjekt benutzt wird.

**13.** Arzneimittelabgabezusammensetzung zur Verwendung nach Anspruch 9,
wobei das Verfahren als Begleittherapie zusätzlich zu einer oder mehreren anderen dopaminergen Medikationen verwendet wird, und/oder
wobei das wenigstens eine Polymer ein thermoplastisches Elastomer ist, das Polymere auf der Basis von Polyurethan, Polymere auf Polyetherbasis, Polymere auf Polysilikonbasis, Polymere auf Polycarbonatbasis oder Kombinationen davon umfasst, und/oder
wobei das wenigstens eine Polymer ein Polyurethan auf Polyetherbasis umfasst.

**14.** Subkutanes Abgabesystem nach Anspruch 1, das Folgendes umfasst:

ein elastomeres Reservoir-Implantat, das wenigstens eine diskrete feste Darreichungsform umfasst, die von einem polymeren Rate regulierenden Hilfsstoff umgeben ist,
wobei die wenigstens eine diskrete feste Darreichungsform Rasagilin-Hemitartrat umfasst,
wobei das subkutane Abgabesystem die Freisetzung des Rasagilins mit einer Elutionsrate vorsieht, die zum Bereitstellen einer therapeutischen wirksamen Menge des Rasagilins einem Subjekt mit einer Null-Ordnung oder Pseudo-Null-Ordnung für eine Zeitperiode von wenigstens einem Monat geeignet ist.

**15.** Kit zum subkutanen Platzieren einer Arzneimittelabgabezusammensetzung, der Folgendes umfasst:

eine Arzneimittelabgabezusammensetzung auf Reservoir-Basis nach Anspruch 1, umfassend einen polymeren Rate regulierenden Hilfsstoff, der ein Reservoir definiert, das wenigstens eine diskrete feste Darreichungsform

enthält, die Rasagilin-Hemitartrat umfasst; und
einen Implantator zum Einführen der Arzneimittelabgabezusammensetzung auf Reservoir-Basis unter die Haut.

## Revendications

1. Composition pour la délivrance d'un médicament comprenant :

   un excipient contrôlant la vitesse d'élution du médicament comprenant un polymère élastomère qui définit un réservoir et
   le réservoir contenant au moins une forme pharmaceutique solide discrète comprenant de l'hémitartrate de rasagiline,
   où la composition pour la délivrance d'un médicament est sous une forme pharmaceutique implantable.

2. Composition pour la délivrance d'un médicament selon la revendication 1, où le polymère élastomère est un élastomère thermoplastique comprenant des polymères à base de polyuréthane, des polymères à base de polyéther, des polymères à base de polysilicone, des polymères à base de polycarbonate ou des combinaisons de ceux-ci et/ou où
   le polymère élastomère comprend un polyuréthane à base de polyéther.

3. Composition pour la délivrance d'un médicament selon la revendication 1, où le polyuréthane à base de polyéther est un polyuréthane à base de polyéther aliphatique comprenant un poly(oxyde de tétraméthylène) et le 4,4'-diisocyanatodicyclohexylméthane ($H_{12}MDI$) et le 1,4-butanediol polymérisés.

4. Composition pour la délivrance d'un médicament selon la revendication 3, où le polyuréthane à base de polyéther présente une dureté Shore inférieure à 87A.

5. Composition pour la délivrance d'un médicament selon la revendication 1, où le polymère élastomère comprend un polyétheramide et/ou
   où la au moins une forme pharmaceutique solide discrète est cylindrique.

6. Composition pour la délivrance d'un médicament selon la revendication 1, où le réservoir contient 5-10 formes pharmaceutiques solides discrètes.

7. Composition pour la délivrance d'un médicament selon la revendication 6, où les formes pharmaceutiques solides discrètes comprennent d'environ 200 mg à environ 500 mg d'hémitartrate de rasagiline.

8. Composition pour la délivrance d'un médicament selon la revendication 1, où l'excipient contrôlant la vitesse d'élution du médicament a une forme cylindrique et/ou
   où la au moins une forme pharmaceutique solide discrète comprend au moins un activateur de sorption sélectionné dans le groupe consistant en la croscarmellose sodique, le carboxyméthylamidon sodique, le glycolate d'amidon sodique, des dérivés sodique de l'acide acrylique, le sulfate de chondroïtine, l'acide poly(glutamique), l'acide poly(aspartique) et des combinaisons de ceux-ci.

9. Composition pour la délivrance d'un médicament pour une utilisation en tant que médicament dans une méthode de traitement de un ou plusieurs symptômes de la maladie de Parkinson, qui comprend :

   l'implantation d'une composition pour la délivrance d'un médicament basée sur un réservoir chez un sujet pour administrer systémiquement une quantité thérapeutiquement efficace de rasagiline au sujet pendant une période d'au moins un mois,
   où la composition pour la délivrance d'un médicament comprend au moins une forme pharmaceutique solide discrète comprenant de l'hémitartrate de rasagiline entourée par un excipient comprenant au moins un polymère.

10. Composition pour la délivrance d'un médicament pour une utilisation selon la revendication 9,
    où la au moins une forme pharmaceutique solide discrète comprend :

    75-97 % en poids d'hémitartrate de rasagiline rapportés au poids total de la au moins une forme pharmaceutique solide discrète ;

1-25% en poids d'au moins un activateur de sorption rapporté au poids total de la au moins une forme pharmaceutique solide discrète ; et
0-5 % en poids d'un lubrifiant rapportés au poids total de la au moins une forme pharmaceutique solide discrète.

**11.** Composition pour la délivrance d'un médicament pour une utilisation selon la revendication 9,
où la quantité thérapeutiquement efficace de rasagiline est délivrée à une vitesse de pseudo ordre zéro et/ou
où la composition pour la délivrance d'un médicament ne requiert pas une érosion ou une dégradation de l'excipient *in vivo* pour libérer la rasagiline à la quantité thérapeutiquement efficace.

**12.** Composition pour la délivrance d'un médicament pour une utilisation selon la revendication 9,
où la quantité thérapeutiquement efficace de rasagiline est délivrée au sujet dans une plage cible qui va d'environ 100 à environ 1 000 microgrammes/jour et/ou
où la méthode est utilisée en monothérapie dans le traitement des symptômes de la maladie de Parkinson du sujet.

**13.** Composition pour la délivrance d'un médicament pour une utilisation selon la revendication 9,
où la méthode est utilisée en thérapie adjuvante de un ou plusieurs autres médicaments dopaminergiques et/ou
où le au moins un polymère est un élastomère thermoplastique comprenant des polymères à base de polyuréthane, des polymères à base de polyéther, des polymères à base de polysilicone, des polymères à base de polycarbonate ou des combinaisons de ceux-ci et/ou
où le au moins un polymère comprend un polyuréthane à base de polyéther.

**14.** Système de délivrance sous-cutanée selon la revendication 1 qui comprend :

un implant à réservoir élastomère comprenant au moins une forme pharmaceutique solide discrète entourée par un excipient polymère contrôlant la vitesse,
la forme pharmaceutique solide discrète comprenant de l'hémitartrate de rasagiline,
où le système de délivrance sous-cutanée produit la libération de la rasagiline à une vitesse d' élution appropriée pour fournir une quantité thérapeutiquement efficace de rasagiline à un sujet à une vitesse d'ordre zéro ou de pseudo ordre zéro pendant une période d'au moins un mois.

**15.** Kit pour la mise en place sous-cutanée d'une composition pour la délivrance d'un médicament qui comprend :

une composition pour la délivrance d'un médicament basée sur un réservoir selon la revendication 1 comprenant un excipient polymère contrôlant la vitesse qui définit un réservoir contenant au moins une forme pharmaceutique solide discrète comprenant de l'hémitartrate de rasagiline ; et
un dispositif d' implantation pour insérer la composition pour la délivrance d'un médicament basée sur un réservoir sous la peau.

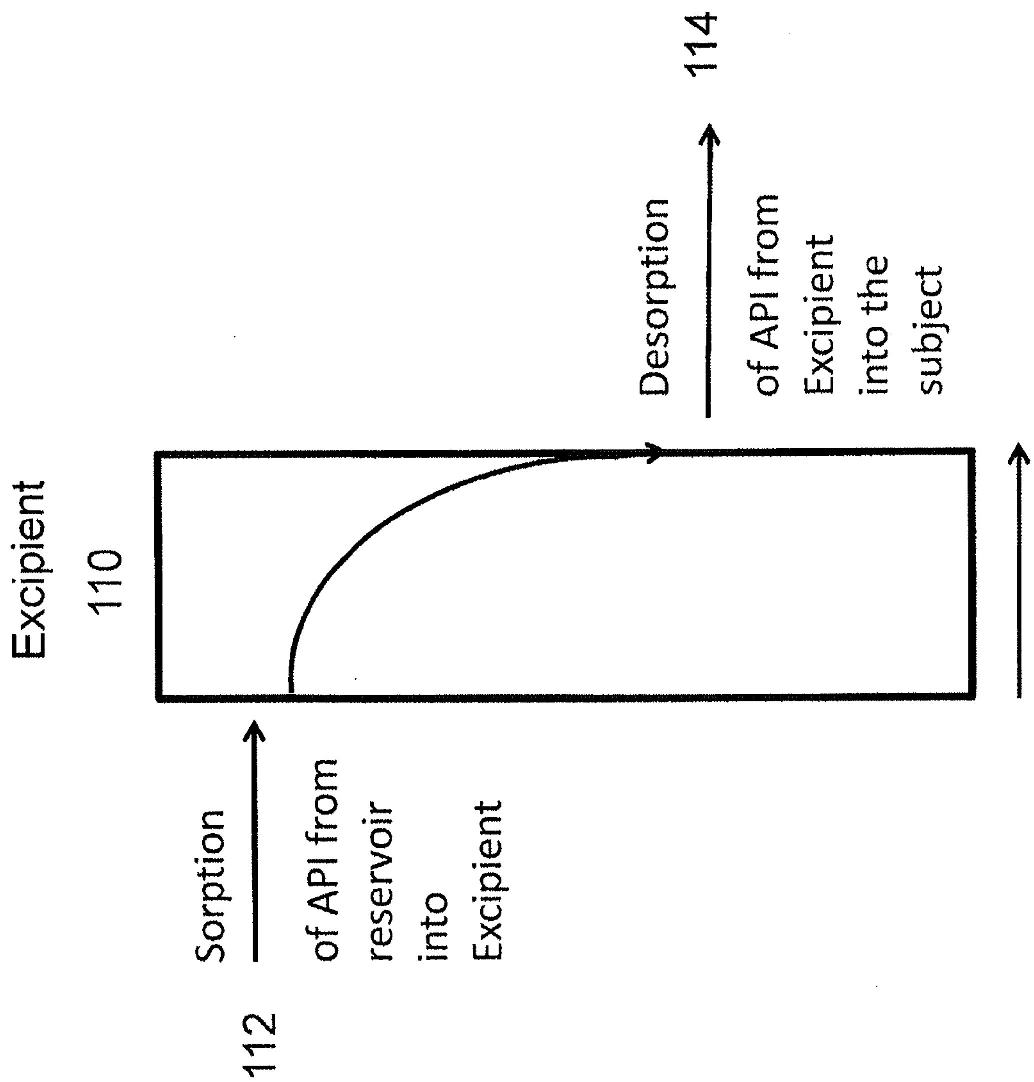
Excipient
110
Sorption
of API from
reservoir
into
Excipient
112
Desorption
of API from
Excipient
into the
subject
114

Figure 1

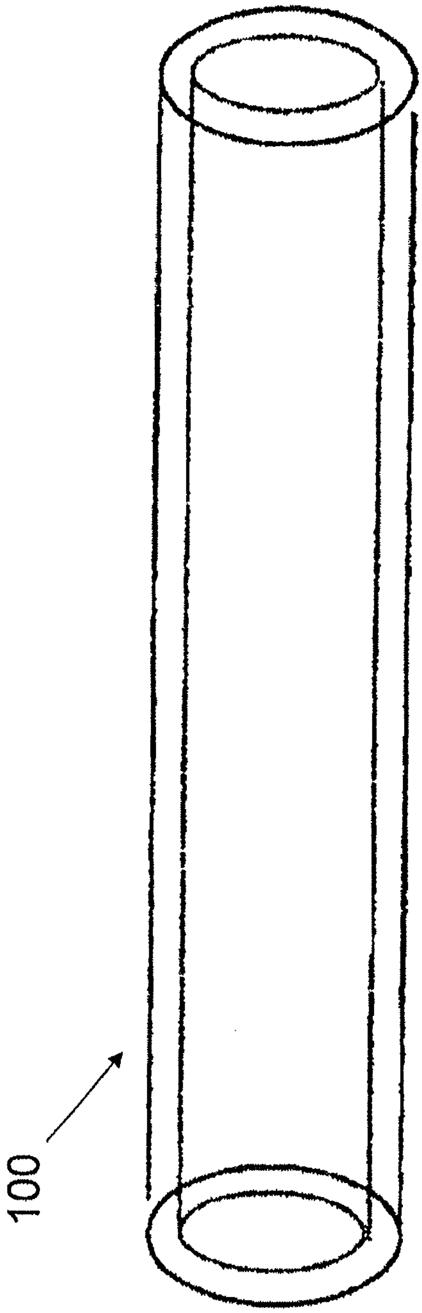


Figure 2

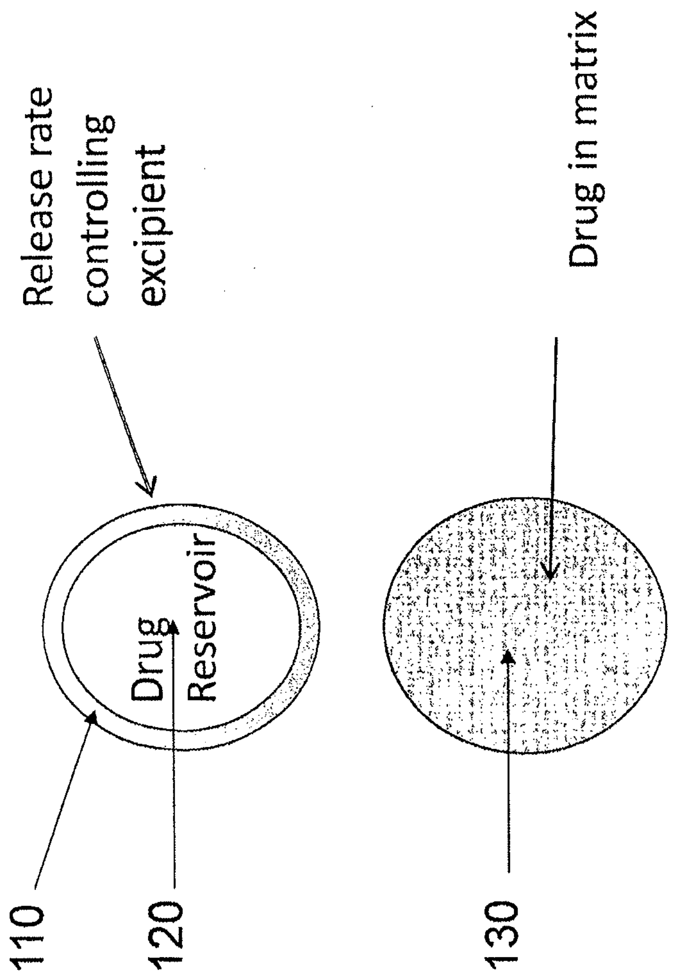
Release rate controlling excipient
Drug Reservoir
Drug in matrix
110
120
130

Figure 3

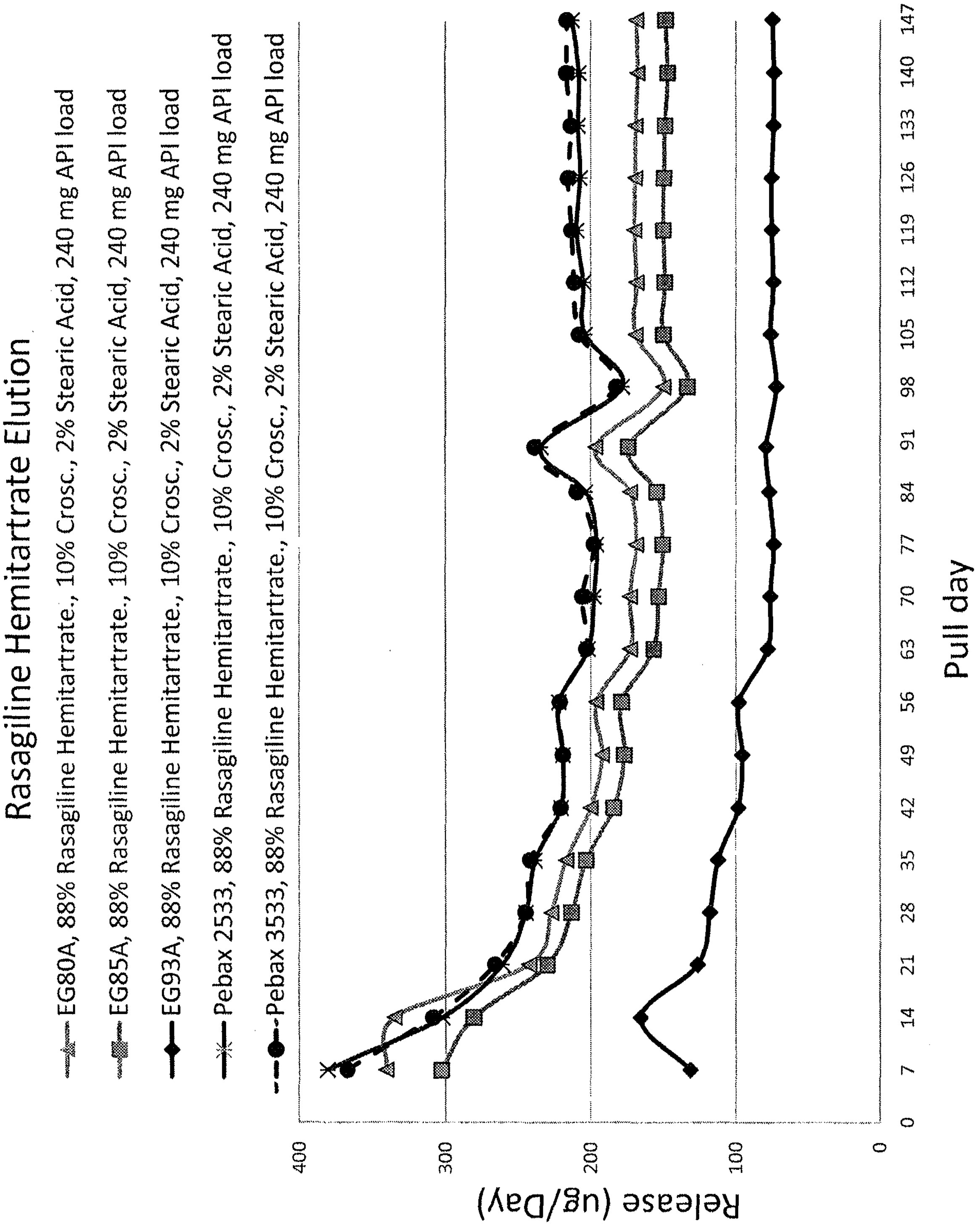
Rasagiline Hemitartrate Elution
EG80A, 88% Rasagiline Hemitartrate., 10% Crosc., 2% Stearic Acid, 240 mg API load
EG85A, 88% Rasagiline Hemitartrate., 10% Crosc., 2% Stearic Acid, 240 mg API load
EG93A, 88% Rasagiline Hemitartrate., 10% Crosc., 2% Stearic Acid, 240 mg API load
Pebax 2533, 88% Rasagiline Hemitartrate., 10% Crosc., 2% Stearic Acid, 240 mg API load
Pebax 3533, 88% Rasagiline Hemitartrate., 10% Crosc., 2% Stearic Acid, 240 mg API load
Release (ug/Day)
400
300
200
100
0
0
7
14
21
28
35
42
49
56
63
70
77
84
91
98
105
112
119
126
133
140
147
Pull day

Figure 4

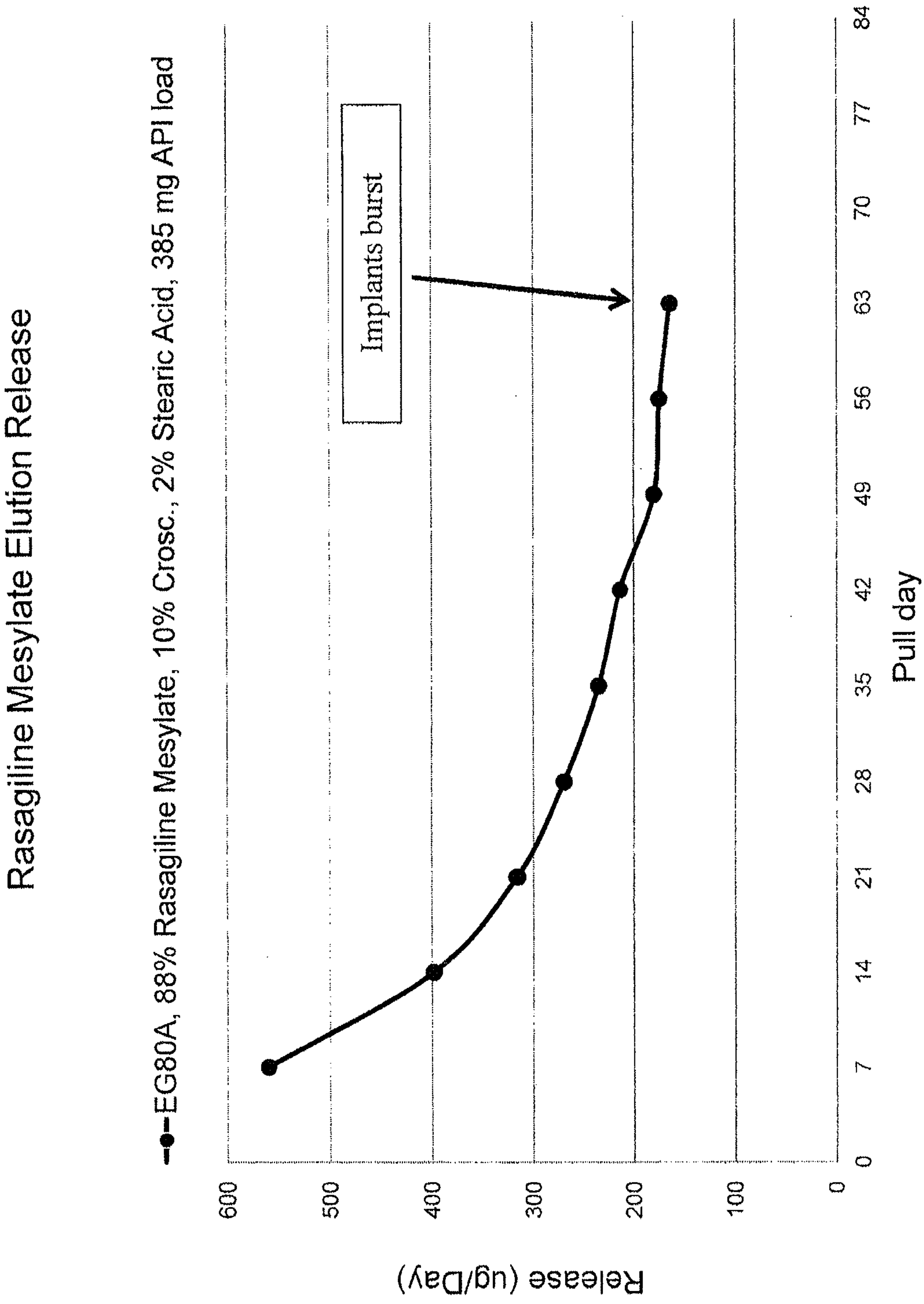
Rasagiline Mesylate Elution Release
EG80A, 88% Rasagiline Mesylate, 10% Crosc., 2% Stearic Acid, 385 mg API load
Implants burst
Release (ug/Day)
600
500
400
300
200
100
0
0
7
14
21
28
35
42
49
56
63
70
77
84
Pull day

Figure 5

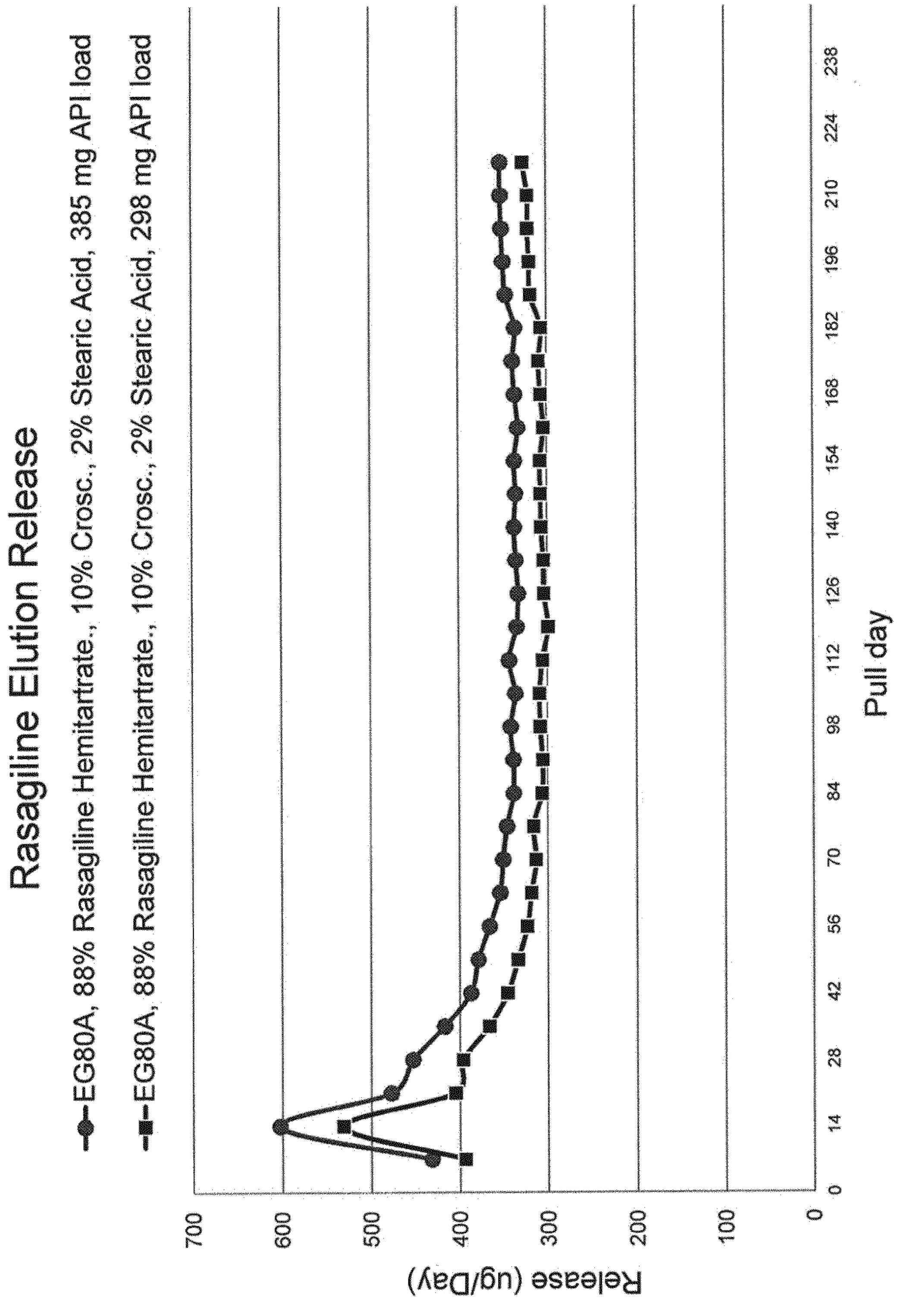
Rasagiline Elution Release
EG80A, 88% Rasagiline Hemitartrate., 10% Crosc., 2% Stearic Acid, 385 mg API load
EG80A, 88% Rasagiline Hemitartrate., 10% Crosc., 2% Stearic Acid, 298 mg API load
Release (ug/Day)
700
600
500
400
300
200
100
0
0
14
28
42
56
70
84
98
112
126
140
154
168
182
196
210
224
238
Pull day

Figure 6

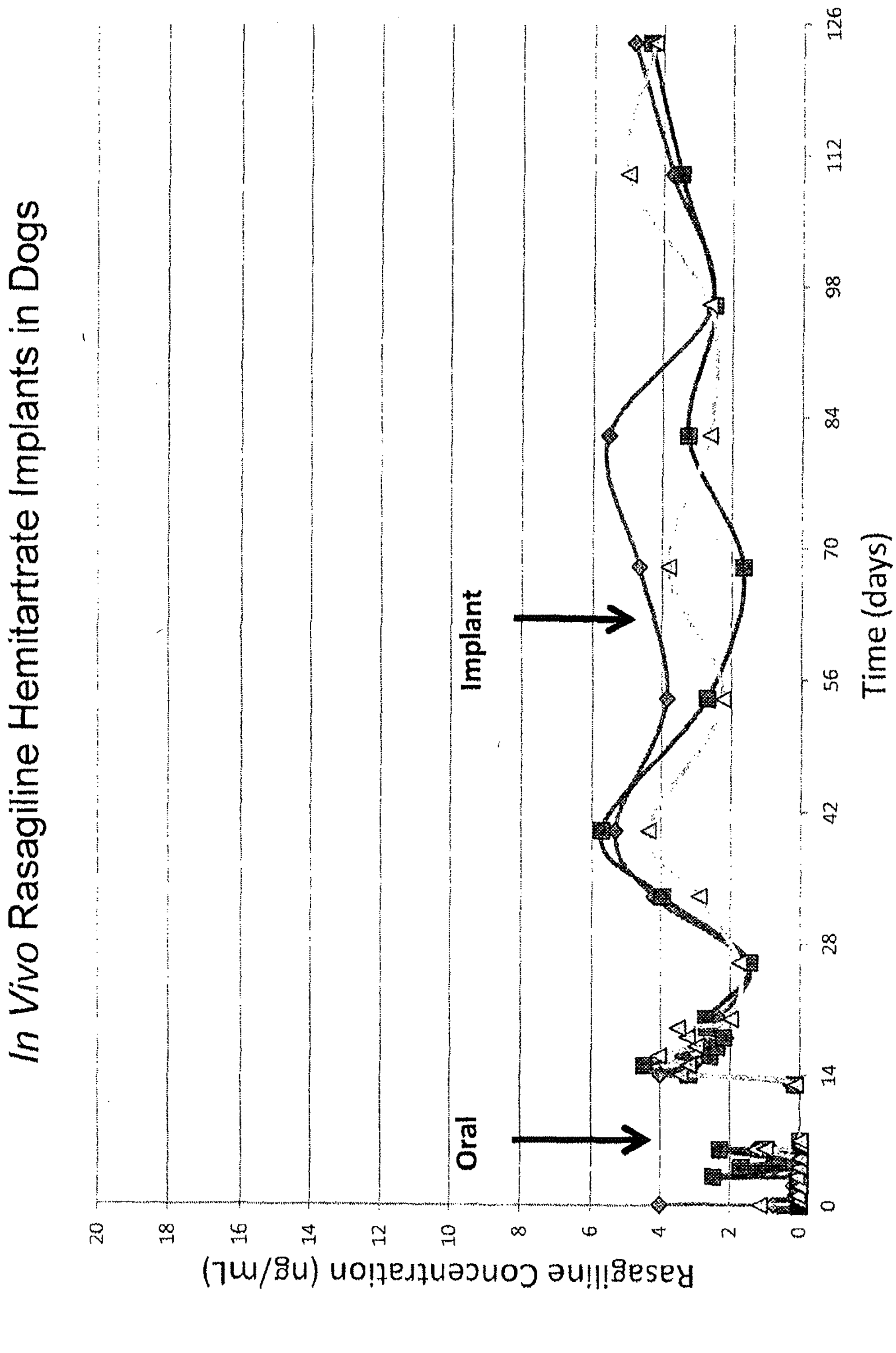
In Vivo Rasagiline Hemitartrate Implants in Dogs
Oral
Implant
Rasagiline Concentration (ng/mL)
0
2
4
6
8
10
12
14
16
18
20
Time (days)
0
14
28
42
56
70
84
98
112
126

Figure 7

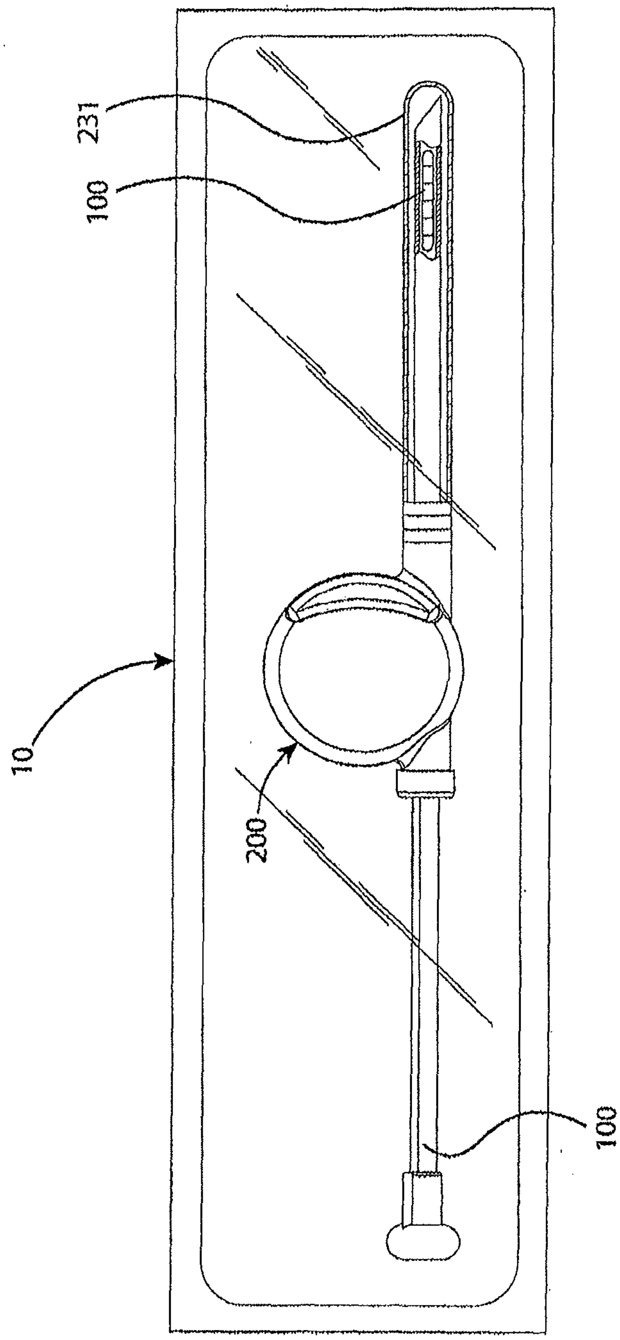


FIG. 8

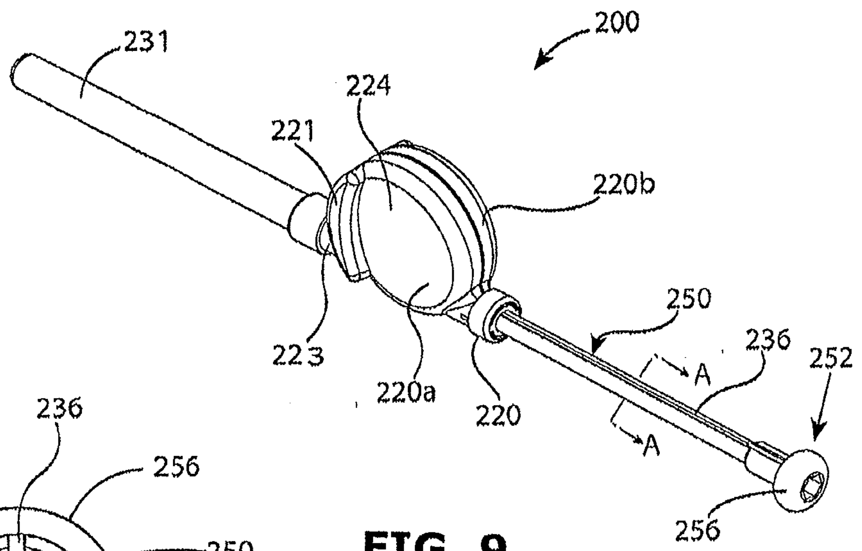


FIG. 9

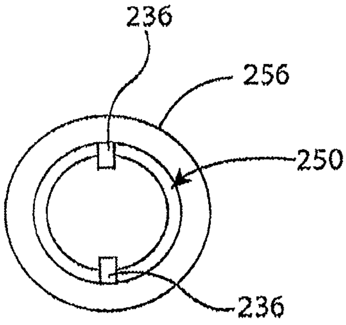


FIG. 9A

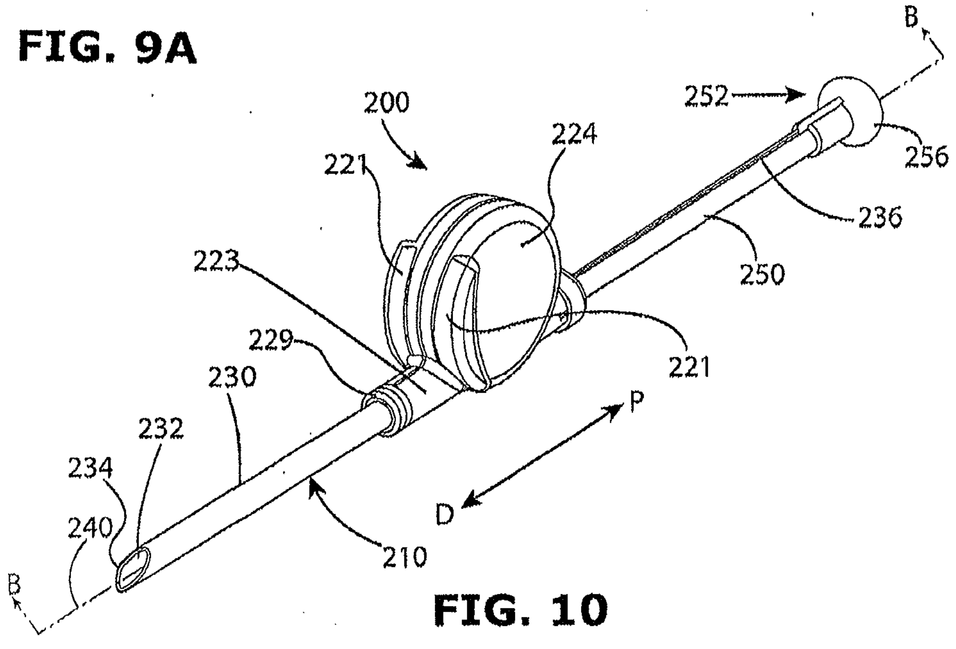


FIG. 10

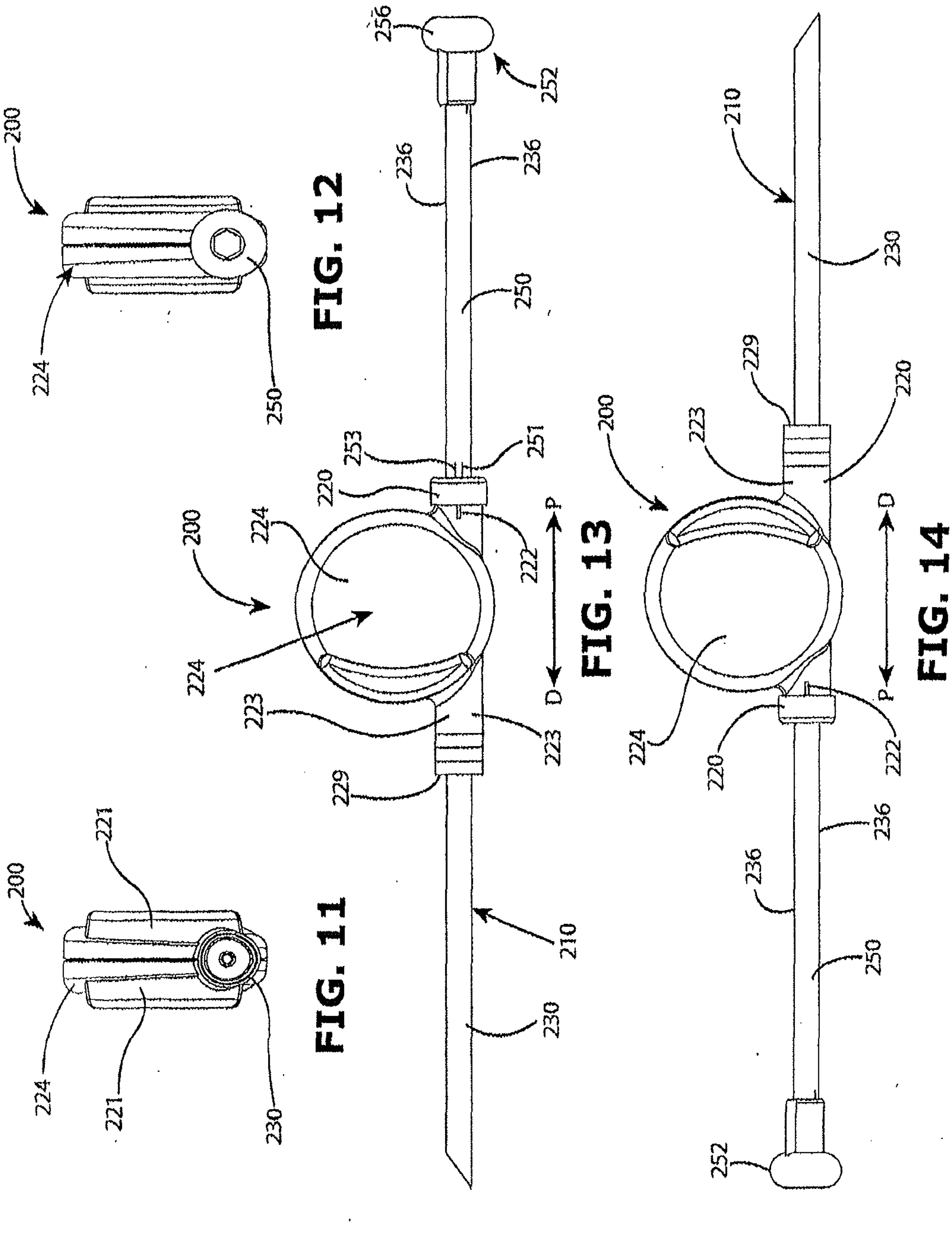


FIG. 11
FIG. 12
FIG. 13
FIG. 14

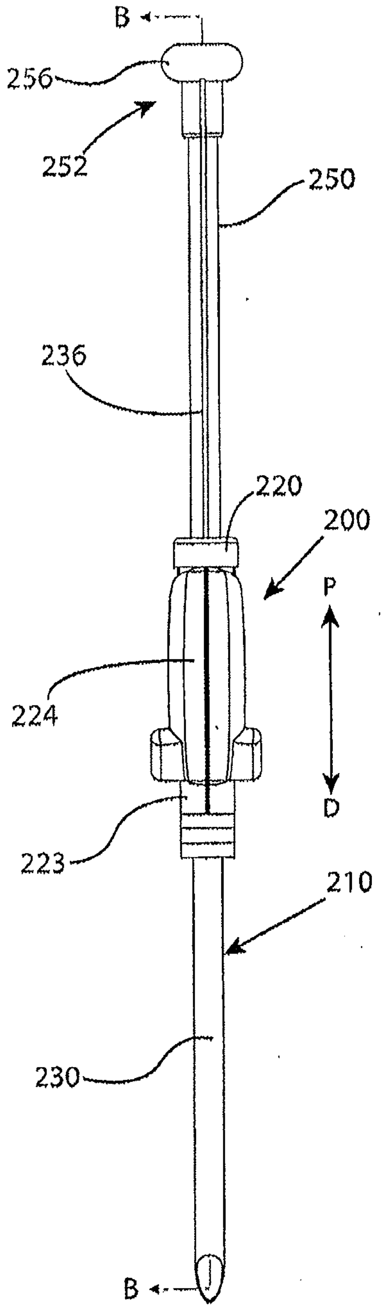
B
256
252
250
236
220
200
P
224
D
223
210
230
B

FIG. 15

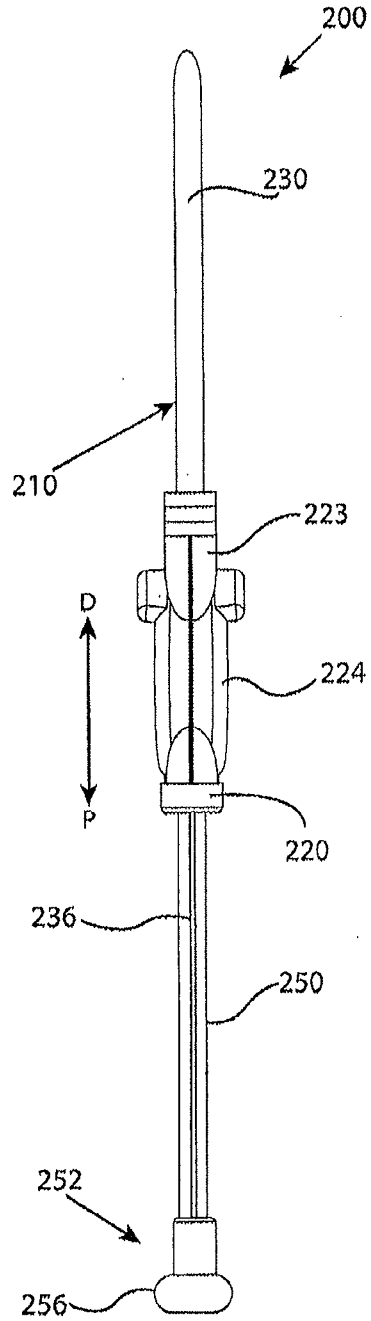
200
230
210
223
D
224
P
220
236
250
252
256

FIG. 16

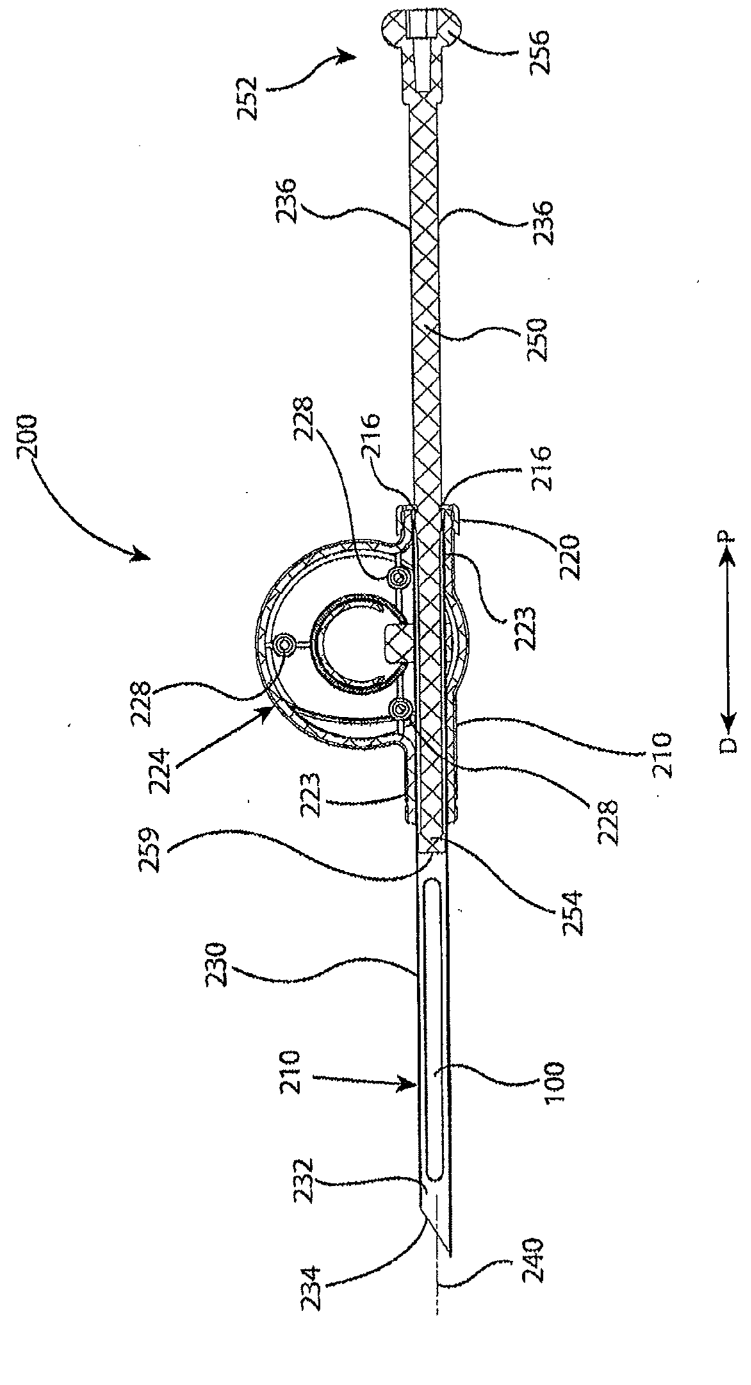
200
252
256
236
236
250
228
216
216
220
223
228
224
228
210
223
228
259
254
230
210
100
232
234
240
D
P

FIG. 17

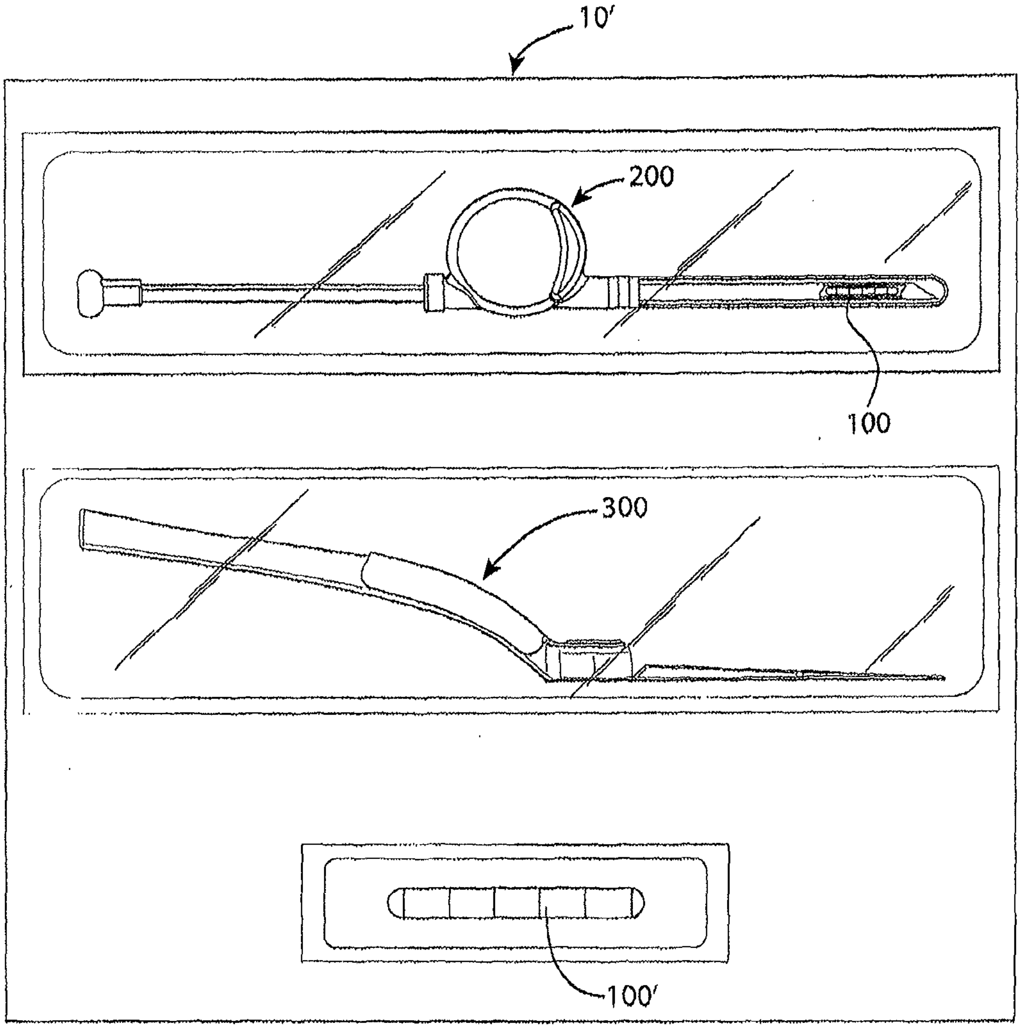


FIG. 18

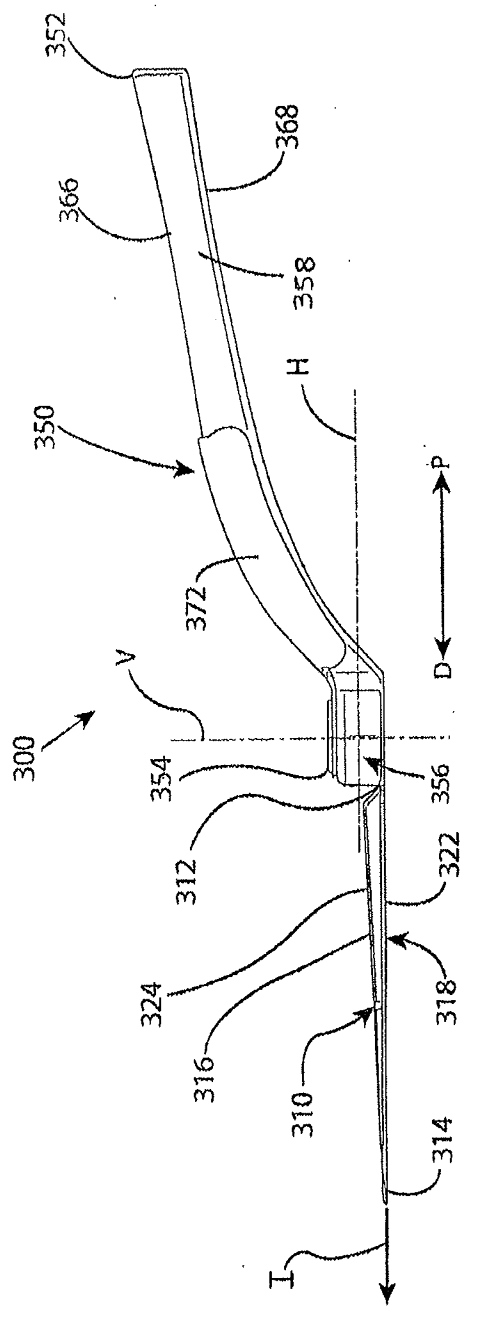
300
350
352
366
368
358
H
V
372
354
312
356
324
322
316
318
310
314
I
D
P

FIG. 19

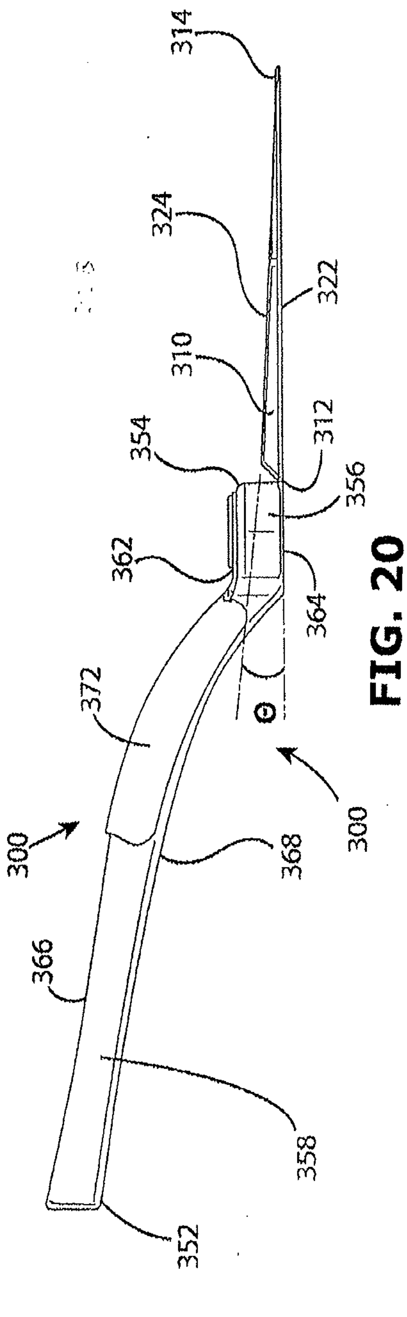
314
324
322
310
312
354
356
362
364
372
Θ
300
368
366
358
352

FIG. 20

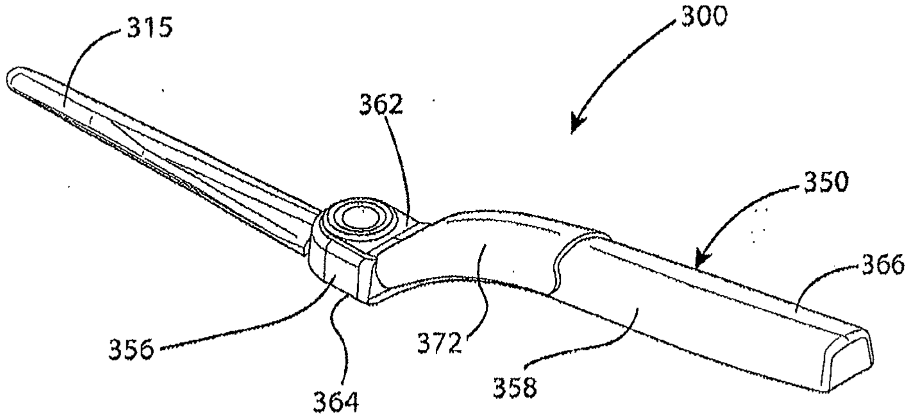


FIG. 21

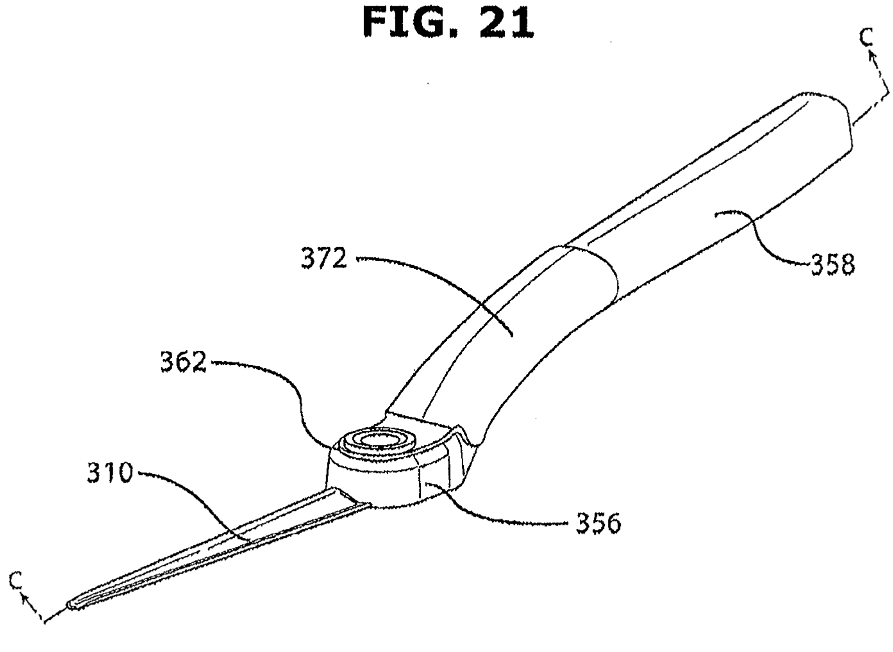


FIG. 22

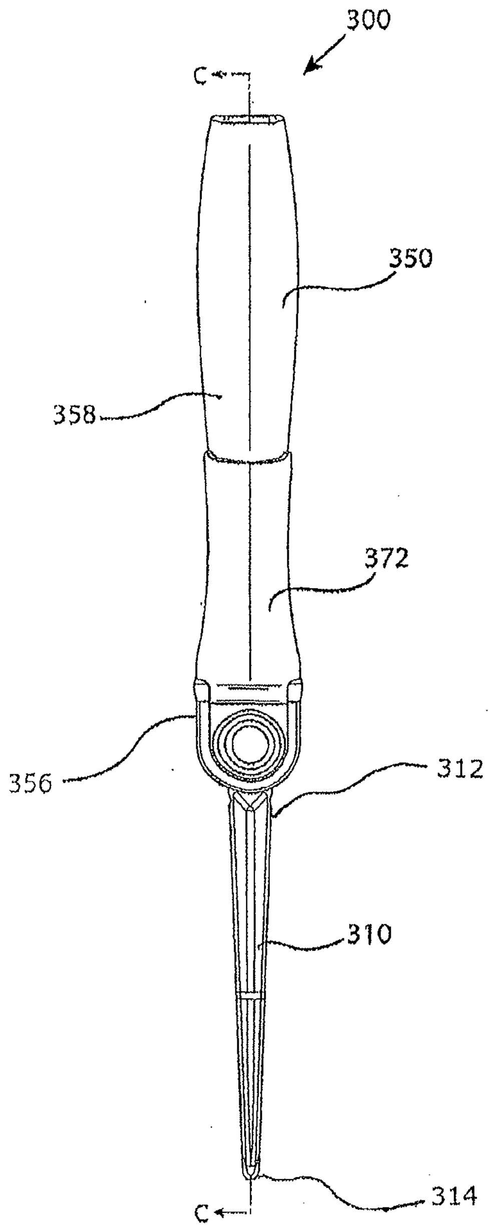


FIG. 23

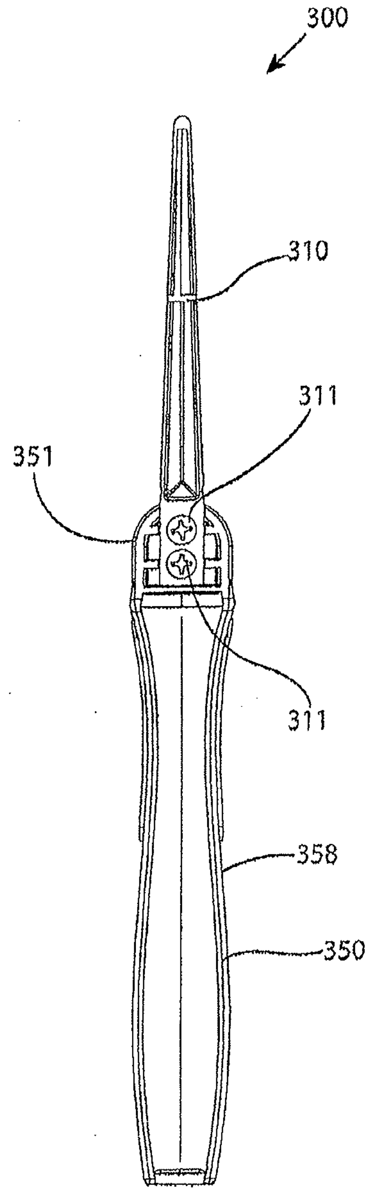


FIG. 24

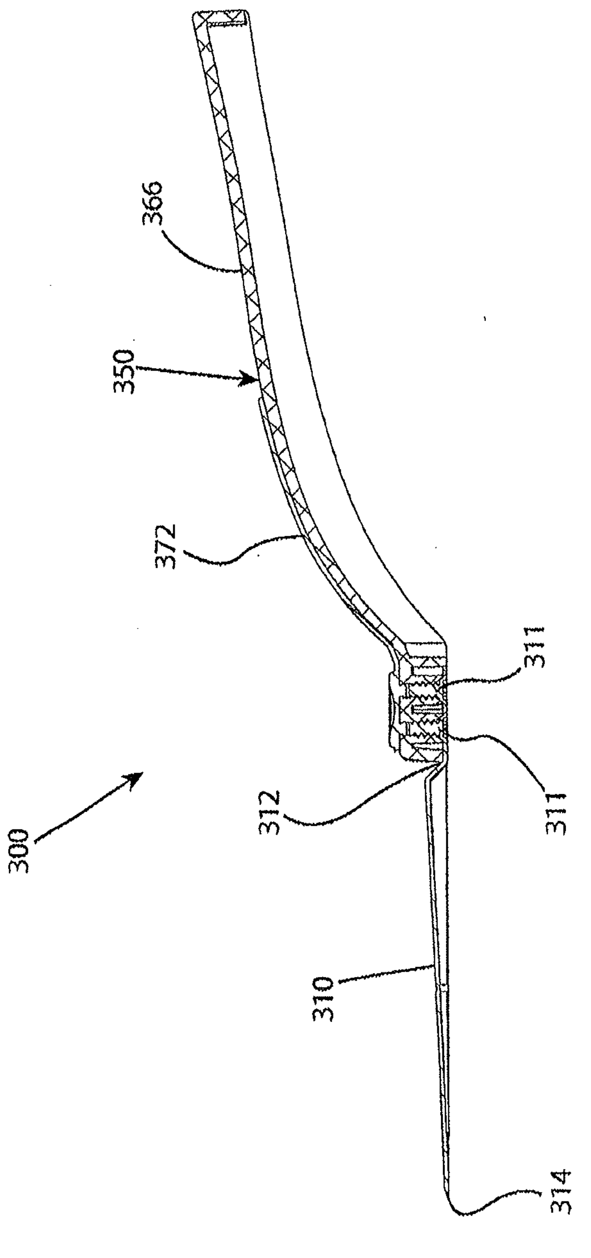


FIG. 25

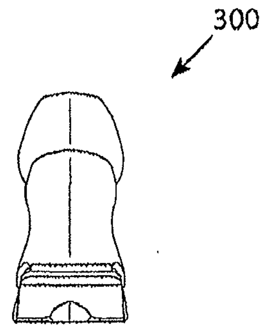


FIG. 26

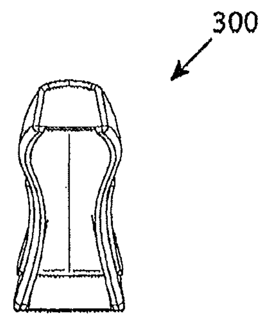


FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7214206 B
- US 7510549 B